# EUROPEAN PATENT SPECIFICATION

(11) **EP 2 155 875 B1**
(45) Date of publication and mention of the grant of the patent: **16.05.2012**
(21) Application number: 08736881.7
(22) Date of filing: 04.04.2008
(51) Int. Cl.: C12N 15/10, C12N 15/52, C12N 15/62, C12N 15/63, C12N 5/10

(54) **NUCLEIC ACIDS AND LIBRARIES FOR USE IN FUNCTIONAL ANALYSIS OF REGULATORY RNAS SUCH AS MICRO-RNAS (MIRNAS)**
NUKLEINSÄUREN UND BIBLIOTHEKEN ZUR VERWENDUNG BEI DER FUNKTIONSANALYSE REGULATORISCHER RNAS, WIE ETWA MIKRO-RNAS (MIRNAS)
ACIDES NUCLÉIQUES ET BIBLIOTHÈQUES POUR L'UTILISATION DANS L'ANALYSE FONCTIONELLE D'ARNS RÉGULATEURS TELS QUE LES MICRO-ARNS (MIARNS)

(30) Priority: 04.04.2007 GB 0706631
(43) Date of publication of application: 24.02.2010
(73) Proprietor: King's College London, London WC2R 2LS (GB)
(72) Inventor: GAKEN, Johannes, Adrianus, London SE22 9JR (GB); MOHAMEDALI, Azim, London SE26 6RG (GB)
(74) Representative: Richards, William John
(86) International application number: PCT/GB2008/001176
(87) International publication number: WO 2008/122770

(56) References cited:
- US-B1- 6 555 370
- "siCHECK(TM) Vectors" TECHNICAL BULLETIN PROMEGA, XX, XX, no. 329, 12 February 2004 (2004-02-12), pages 1-17, XP002292967
- SCHWARTZ F ET AL: "A dominant positive and negative selectable gene for use in mammalian cells" PROCEEDINGS OF THE NATIONAL ACADEMY OF SCIENCES OF USA, NATIONAL ACADEMY OF SCIENCE, WASHINGTON, DC.; US, vol. 88, no. 23, 1 December 1991 (1991-12-01), pages 10416-10420, XP002292587 ISSN: 0027-8424

## Description

### Field of the Invention

The invention relates to materials such as nucleic acids and libraries for use in functional analysis of regulatory RNAs such as microRNAs (miRNAs), and particularly testing of or screening for targets of regulatory RNAs such as 3' untranslated region (UTR) sequences.

### Background to the Invention

MicroRNAs (miRNAs) are now recognized as a novel class of small regulatory RNA molecules that regulate the expression of many genes. They have been shown to mediate angiogenesis, cell adhesion, cell proliferation, survival and play an important role in haematopoiesis. They are produced from primary RNA transcripts (pri-miRNAs) that are processed by the enzyme DROSHA into ~70bp duplexes which are further processed by DICER into ~22bp miRNA duplexes. One strand of the 22bp duplex associates with the RNA-induced silencing complex (RISC) which targets sites within the 3' untranslated region (UTR) of the mRNA resulting in either translational repression, mRNA cleavage or induction of deadenylation. It is currently thought that in humans, the RISC complex acts mainly by inducing specific translational inhibition through binding to the 3' UTR of target mRNA and to a lesser extent degradation of mRNA targets.

MicroRNAs (miRNAs) are a family of mature noncoding small RNAs 21-25 nucleotides in length. They negatively regulate the expression of protein-encoding genes. miRNAs are processed sequentially from primary miRNA (pri-miRNA) precursor transcripts, and regulate gene expression at the post-transcriptional level. The expression of miRNAs is highly specific for tissue and developmental stage, but little is known about how these expression patterns are regulated. More than 541 human miRNA genes have been identified, but recent bioinformatic approaches predict the number to be closer to 1,000. Current estimates suggest that about one-third of human mRNAs appear to be miRNA targets. They have been shown to mediate angiogenesis, cell adhesion, cell proliferation, survival and play an important role in haematopoiesis and cancer.

Due to the partial homology between a miRNA and its target and inhibition of translation instead of mRNA degradation, target identification is a difficult task. Bioinformatic algorithms have been developed for the prediction of miRNA targets based on the "seed" sequence. The main four algorithms predict 101,031 miRNA/target pairs (on average 200 targets per miRNA). Only 0.01 % (12) of these pairs are predicted by all 4 algorithms, 2.8% by 3, 15.4% by 2 and 81.8% by only 1 algorithm. Of the 465 human miRNAs identified, only 57 have 103 experimentally validated target sites in 85 genes.

To date, the role and the specific targets of most miRNAs are largely unknown. This is mainly due to the difficulties in identifying targets because, contrary to short interfering RNA (siRNA), miRNA binding is only partially due to homology with the target. Furthermore, the inhibition of translation precludes mRNA expression array studies for target discovery.

To obtain better insight into the function of miRNAs, much effort has been put in the computational identification of miRNA targets using various algorithms (e.g. miRBase (Sanger institute, http://microma.sanger.ac.uk/sequences/), TargetScan (Whitehead Institute for Biomedical Research, http://www.targetscan.orgn and PicTar (New York University, http://pictar.bio.nyu.edu/)). However, the drawbacks of these predictions are that they each generate a substantial number of false positives. Furthermore, the predictions are likely to be inherently biased as they are mostly based on the knowledge obtained from the very few known miRNA: target interactions, a statistically very small sample size which almost certainly leads to a skew on the predictions.

The prior art study of miRNA gene regulation lacks the necessary tools for target identification and validation, particularly regarding functional studies.

siRNAs are known to have catalytic effects and can break down mRNAs. Consequently, siRNAs can be studied by using expression pattern array analysis before and after adding siRNAs. However, since most miRNAs do not have catalytic activity leading to the breakdown of mRNAs, these types of analysis cannot be applied to the study of miRNAs.

Another theory about miRNA function in the prior art is that they prevent extension of the peptide. In this scenario, it would be necessary to look at the protein product in order to analyse miRNA behaviour.

Prior art techniques for miRNA detection have been based on miRNA arrays. These can only be produced with the knowledge of the sequence of the miRNA itself. Furthermore, attempts to study these phenomena have been made using real time PCR for specific miRNAs. However, once again, this type of analysis relies on knowing the precise miRNA sequence.

To obtain better insight into the function of miRNAs, much effort has been put in the computational identification of miRNA targets using various algorithms. However, the drawbacks of these predictions are that they all generate a substantial number of false positives and may be biased as they are mostly based on the knowledge obtained from the few known miRNA:target interactions. Thus, in this field, finding candidate miRNAs is straightforward by computational techniques. However, computational techniques for finding miRNAs suffer from drawbacks such as being inherently biased towards the small number of miRNAs which have in fact been experimentally verified. Since the number of verified miRNAs is very small, the pool of verified miRNA sequences from which conserved motifs or domains can be drawn is correspondingly small. Firstly, this makes it difficult to extrapolate from overlap between the small numbers of known sequences to a wider pool of candidate miRNAs. Secondly, in any statistically small sample from a large overall group there will be an inherent statistical bias by chance. Thus, since the number of miRNAs upon which the computational predictions are based is very small, it is almost certain that a strong statistical bias exists in the predictions.

Furthermore, considering the four principal prediction algorithms, only 0.01% of miRNA/target pairs are predicted by each of the algorithms. Indeed, more than 80% of the pairs are predicted by only one of the algorithms. Thus, accurate identification or validation of miRNA/target pairings is a problem in the art.

A key difficulty in the field is the finding of a target for an miRNA. This is especially difficult since it is known that miRNA targets are not necessarily identical in sequence to the miRNA sequence itself.

A prior art technique which attempts to study or to quantify miRNA action is Ambion Inc's luciferase assay. This involves the cloning of a target and combination with the candidate miRNA, followed by a luciferase assay designed to read out any effect, using plasmid from Ambion: pMIR-REPORT™, cat no. AM5795. Firstly, as will be appreciated, it is typically necessary to know the target or candidate target before this type of analysis can be conducted. Secondly, each individual clone needs to be treated separately since there is no way of separating those harboring nucleic acid of interest from those which do not in a screening type setting.

Another way of analysing the effects of miRNA is by the use of 2D gels to study protein expression patterns. In this scenario, the 2D expression patterns of various proteins are compared between an miRNA treatment and a non miRNA treatment. However, the sensitivity of this technique is very low. It is very likely that not all proteins are detected by this rather crude methodology. Indeed, it is estimated that only approximately 10% of expressed proteins show up in 2D gel type protein expression analysis. Clearly, this approach is not sensitive enough for a meaningful study of miRNA action.

Furthermore, as noted above, since miRNAs do not degrade the target RNA in the same manner that siRNAs do, it is also not possible to study miRNA action by monitoring mRNA levels.

WO2004/097042 discloses an siRNA selection method. siRNAs exhibit 100% identity to their target sequences. The clones used comprise only one marker per transcript. The method is used to select siRNA directed to cloned cDNA.

"siCHECK™ Vectors" Technical Bulletin Promega No. 329 (12 Feb 2004) pages 1-17 discloses certain vectors for optimisation of RNA interference (RNAi). In particular, the psiCHECK™-1 and psiCHECK™-2 vectors are disclsoed. These comprise synthetic *Renilla* luciferase as a reporter gene. The document also discloses tables of restriction enzymes having sites therein, maps of the vectors, and buffers for effective luciferase luminescence.

The prior art suffers from shortcomings as noted above. Furthermore, there is no functional assay for target discovery in the field of miRNA in existence in the prior art. In addition, there are examples which expose limitations of the computational models. For example, LED7 is an miRNA from *C.elegans.* This gene (known as "lethal 7") knocks out various genes and leads to apoptosis in those cell lineages in which it is expressed. Applying the computational models, it is possible to identify predicted sequences which LED7 should bind to. However, many of these predicted targets are shown experimentally not to bind to LED7 at all. By contrast, the ETWK3 gene has been studied. In the course of this study, the miRNA named miR143 has been proven to be a bona fide target of ETWK3. However, miR143 is not predicted by all of the computational models noted above, but at best only by a proportion of them. Therefore, in addition to predicting targets which are not in fact bound by the miRNA, computational models also do not predict bona fide miRNA pairings. Therefore, it can be appreciated that these computational systems in the art have numerous serious problems and drawbacks associated with them.

The present invention seeks to overcome problems associated with the prior art.

### Summary of the Invention

The present inventors have advantageously designed a new system which enables a functional assay for regulatory RNA such as miRNA action. The present invention advantageously combines a selectable genetic marker with a cloning system into which candidate 3-prime UTR's can be inserted. In this way, it becomes possible to study the effects of various miRNAs both in a positive and in a negative fashion and the expression of particular RNAs. The key concept is that the RNAs which are being studied (the candidate 3-prime UTR's or target sites for miRNA action) are directly coupled to the coding sequence for the positive and/or negative selectable marker.

Therefore, by following the selectable marker or markers, a direct functional readout of the effect of particular miRNAs and those mRNAs can advantageously be obtained. The present invention is based upon this surprising finding. A key advantage of the invention is that is provides a functional readout at the protein level. Although some regulatory RNAs such as siRNA produce cleavage of the target RNA, which allows assay at the RNA level for example by monitoring RNA levels or cleavage, other regulatory RNAs such as miRNAs do not produce this effect. By assaying the effects at the protein level as described herein, numerous regulatory RNA types may be studied functionally, which is an advance compared to prior art techniques.

In a first aspect the invention provides a nucleic acid comprising the following contiguous elements arranged in the 5 prime to 3 prime direction;
a) a promoter;
b) at least two selectable markers;
c) a cloning site for receipt of a nucleic acid segment, said segment comprising a candidate regulatory RNA target sequence; and
d) a poly adenylation signal,
said elements arranged such that a transcript directed by said promoter comprises said at least two selectable markers, said candidate regulatory RNA target sequence, and said poly adenylation signal in that order.

Suitably the nucleic acid comprises DNA; for example a DNA plasmid. When the nucleic acid comprises DNA, references to RNA target sequences, microRNA and similar are to be understood according to convention i.e. that they define the nucleotide sequence which is specified and do not necessarily require that the nucleic acid is RNA (or a DNA-RNA hybrid). The skilled reader will therefore understand the nucleotide sequence to comprise T or U at the appropriate position as dictated by the nature of the nucleic acid as is conventional in the art.

It is an important feature that the elements are arranged such that a transcript (a single transcript) directed by said promoter comprises said selectable markers, said candidate regulatory RNA target sequence, and said poly adenylation signal in that order i.e. as a single 'fused' RNA transcript. Known plasmids for unconnected applications do not admit fusion of the transcript in this manner, for example conventional cDNA libraries do not direct such fused transcripts. This is a particular advantage of the invention.

Suitably said candidate regulatory RNA target sequence is a candidate microRNA (miRNA) target sequence or a candidate short interfering RNA (siRNA) target sequence. Suitably said candidate regulatory RNA target sequence is a candidate microRNA (miRNA) target sequence.

The term 'selectable marker(s)' used in connection with nucleic acids of the invention has its ordinary meaning in the art and suitably refers to a nucleic acid comprising an open reading frame encoding a polypeptide selectable marker i.e. a polypeptide which confers a selectable property or activity.

Suitably the nucleic acid further comprises a stop codon located between said selectable marker and said cloning site. Suitably said stop codon is a stop box comprising stop codons in each of the three forward frames.

The selectable marker(s) may be for positive selection.

The selectable marker(s) may be for negative selection.

Suitably the nucleic acid may further comprise (e) a transcriptional terminator signal. It is considered that the polyadenylation signal will typically be sufficient for higher eukaryotic such as mammalian applications of the invention, but if the invention is applied in lower eukaryotes such as unicellular eukaryotes or even prokaryotes then a transcriptional terminator may provide advantageous extra control of RNA transcription.

The selectable marker suitably comprises two or more selectable markers, suitably two selectable markers. Suitably said two or more selectable markers are provided as a single polypeptide or open reading frame (i.e. a 'fusion protein'). Thus suitably said two selectable markers are provided as an open reading frame encoding a single polypeptide comprising said two selectable markers. Suitably said selectable markers comprise at least one marker for positive selection and at least one marker for negative selection. Suitably said selectable marker is an HSVTK/PURO fusion protein.

Suitably said cloning site is a directional cloning site.

Suitably said cloning site has inserted therein a nucleic acid segment comprising a 3 prime UTR or a candidate 3 prime UTR. In another aspect, the invention provides a 3 prime UTR library, said library comprising a plurality of said nucleic acids. Suitably said candidate miRNA target sequences are comprised by cDNA's. Suitably said candidate miRNA target sequence is less than 6kb. Suitably said candidate miRNA target sequence is approximately 2kb.

Suitably said cDNA's are brain cDNA's, testes cDNA's or are cDNA's from acute myeloid leukaemia cells.

The invention also provides cell(s) comprising a nucleic acid as described above, or comprising libraries as described above.

In another aspect, the invention provides a population of cells, said cells together harbouring at least part of a library as described above.

In another aspect, the invention provides a method of making a 3 prime UTR library comprising providing a nucleic acid as described above, and inserting into said cloning site a nucleic acid comprising a 3 prime UTR or a candidate 3 prime UTR.

In another aspect, the invention provides a method of making a 5 prime UTR library comprising providing a nucleic acid as described above, and inserting into said cloning site a nucleic acid comprising a 5 prime UTR or a candidate 5 prime UTR.

In another aspect, the invention provides a vector comprising a nucleic acid as described above. The vector may be any nucleic acid based vector such as a plasmid vector, transposon vector, viral or retroviral vector, or other vector. Suitably the vector is a plasmid vector. The vector is suitably provided with 'shuttle' elements allowing propagation and/or amplification in host organisms. Suitably said shuttle elements are for propagation in *E.coli* cells and include an *E.coli* origin of replication.

In another aspect, the invention provides a method for identifying a miRNA target sequence comprising the steps of
(a) introducing a nucleic acid as described above comprising a candidate miRNA target sequence into a host cell;
(b) selecting host cell(s) expressing at least one selectable marker of said nucleic acid;
(c) introducing at least one miRNA of interest to said host cell(s) of (b), and
(d) assaying for expression of at least one selectable marker of said nucleic acid in the cells of (c),
wherein if the cells of (c) do not show expression of at least one selectable marker then the candidate miRNA target sequence is identified as a miRNA target sequence.

In another aspect, the invention provides a method for identifying an miRNA active against a miRNA target sequence comprising the steps of
(a) introducing a nucleic acid as described above comprising said miRNA target sequence into a host cell;
(b) selecting host cell(s) expressing at least one selectable marker of said nucleic acid;
(c) introducing at least one miRNA of interest to said host cell(s) of (b), and
(d) assaying for expression of at least one selectable marker of said nucleic acid in the cells of (c),
wherein if the cells of (c) do not show expression of at least one selectable marker then the miRNA of interest is identified as an miRNA active against said miRNA target sequence.

Step (d) may comprise selecting against cells which express at least one selectable marker.

Step (d) may comprise selecting for cells which do not express at least one selectable marker.

In another aspect, the invention provides a method for identifying an inhibitor of a regulatory RNA comprising the steps of
(a) introducing at least one regulatory RNA of interest into a host cell;
(b) introducing a nucleic acid as described above comprising a candidate RNA target sequence into said host cell;
(c) selecting host cell(s) which do not show expression at least one selectable marker of said nucleic acid;
(d) introducing to said host cells a test substance or nucleic acid
(e) assaying for expression of at least one said selectable marker in the cells of (d);
wherein if the cells of (d) show expression of at least one selectable marker then the test substance or nucleic acid is identified as inhibiting said regulatory RNA.

In another aspect, the invention provides a method for identifying a regulatory RNA target sequence comprising the steps of
(a) introducing a nucleic acid as described above comprising a candidate regulatory RNA target sequence into a host cell;
(b) selecting host cell(s) expressing at least one selectable marker of said nucleic acid;
(c) introducing at least one regulatory RNA of interest to said host cell(s) of (b), and
(d) assaying for expression of at least one selectable marker of said nucleic acid in the cells of (c),
wherein if the cells of (c) do not show expression of at least one selectable marker then the candidate regulatory RNA target sequence is identified as a regulatory RNA target sequence.

Suitably said regulatory RNA is a siRNA and said candidate regulatory RNA target sequence is a candidate siRNA target sequence.

In another aspect, the invention provides a method as described above further comprising the step of comparing the target sequences identified to known target sequences of the regulatory RNA of interest, thereby identifying new target sequences of said regulatory RNA.

In another aspect, the invention provides a nucleic acid as described above wherein said nucleic acid comprises the nucleic acid sequence of one or more of SEQ ID NO: 1, SEQ ID NO:2, SEQ ID NO:3, SEQ ID NO:4, SEQ ID NO:5, SEQ ID NO:6 or SEQ ID NO:7.

In another aspect, the invention provides a nucleic acid as described above wherein said nucleic acid is selected from plasmids p3'UTR3, p3'UTRTKPuro, p3'UTRHyTK or p3'UTRTKzeo.

### Detailed Description of the Invention

The invention advantageously provides a functional assay for microRNA target discovery and validation. It will be understood that microRNA is one class of regulatory RNAs, such as small regulatory RNAs. Other classes of small regulatory RNA may also be addressed in embodiments set out herein. In particular, small interfering RNA (siRNA) may be substituted for microRNA. Both miRNA and siRNA applications may even be combined. For convenience, the invention is described with most reference to miRNA as the regulatory RNA.

The term 'seed sequence' is well known in the art and typically refers to the 5' end of the regulatory RNA (e.g. siRNA or miRNA). This typically refers to the 6 or 7 bases at the 5' end of the regulatory RNA. These typically are a 100% match to the target sequence.

The term `3' UTR' literally means 3 prime untranslated region. This is the region of a mRNA which is not translated and is often a target of translational regulation for example by miRNAs. The term is often used within with the broader term 'miRNA target sequence' herein since it is possible that a miRNA target sequence may not have been derived from, or experimentally demonstrated to be, a 3' UTR e.g. if the miRNA target sequence has been generated or derived from a non-mRNA source. Typically most or all miRNA target sequences are found in 3' UTRs. However, clearly miRNA target sequences may be derived from other locations for example from the genome as a whole, or may even be artificially created by generating a library or random or semi-random sequences which may comprise miRNA target sequences. Thus, it must be borne in mind that the invention applies generally to miRNA target sequences, and that for convenience these are often referred to as 3' UTR's herein, but that said target sequences or candidate target sequences may in fact be derived from one or more sources which are distinct from actual experimentally defined 3' UTRs. Suitably the miRNA target sequence is, or is derived from, a 3' UTR.

The term 'cloning site' has its ordinary meaning in the art. In particular it refers to a nucleic acid element or sequence which permits digestion of the nucleic acid by a restriction enzyme or similar catalyst to allow insertion of nucleic acid into said digested site. Examples of cloning sites are multiple cloning sites ('MCS') which feature nucleic acid sequence comprising recognition sites for multiple nucleic acid restriction enzymes thereby allowing alternative cloning strategies into a single cloning site. Suitably the cloning site of the invention comprises nucleic acid sequence recognisable by at least one restriction enzyme, suitably a restriction enzyme allowing directional cloning, suitably SfiI. Thus, in one embodiment the cloning site is simply a SfiI recognition site.

The coding sequence of polypeptides to be expressed according to the present invention may advantageously be codon optimised for the target cell (host cell) in which expression is to take place. In particular, suitably the selectable markers are codon optimised to the cells in which selection is to take place. Suitably codon optimisation is to human criteria for human cells.

### Advantages of the Invention

Prior art techniques for analysing miRNA action are based upon the use of luciferase. Luciferase is a protein whose activity can be measured by monitoring luminosity or light emitted. Luciferase does not afford any positive or negative selection. Using a luciferase based system, it is undoubtedly very labour intensive to screen for the effects of particular miRNAs. Firstly, if this technique was to be applied to 3 prime UTR's or candidate 3 prime UTR's, each would have to be done in a separate treatment. This could involve anything up to 40-100,000 separate experiments or treatments. Clearly, this is a very cumbersome and expensive procedure to perform. By contrast, according to the present invention, miRNA action can be assessed using genetic selection techniques. This advantageously allows cells expressing certain selectable markers to be selected, and for the effects of miRNA (whether positive or negative) to be directly genetically selected without resorting to any luminescence assay. In addition to avoiding time consuming luminescence assays, the present invention offers the further advantage of being able to handle multiple analyses in parallel since only cells harbouring (or expressing) certain pre-determined genetic constructs will survive the selection procedures.

In order to better understand this advantage, consider the following illustration. Firstly, according to the present invention cells harbouring a particular genetic construct can be selected in a first step of positive selection. This results in the loss of cells which are not harbouring nucleic acid of interest. Thus, all the surviving cells must by inference (by selection) be harbouring the genetic construct of interest. This first positively selected population of cells can then proceed to the second step of the procedure. In the second step of the procedure, those cells are treated with miRNA, and those cells in which the miRNA affects protein expression of the marker of interest are selected. Thus, by performing this second selection step those cells harbouring a genetic construct which is responsive to the particular miRNA being studied are genetically isolated.

It is an advantage of the invention that a population of cells can be studied by the multiple selective procedure. Indeed, in practical terms, it is an advantage of the invention that a population of cells can be studied in a single dish, which cells individually harbour different genetic constructs. Of course, when studying a large population of cells, or for convenience depending upon the format of the study, multiple dishes may be advantageous, but a key advantage is that the multiple selective procedure allows parallel handling of cells harbouring different genetic constructs at the selection stage, rather than having to handle individual clones separately throughout the procedure. This type of application is clearly not possible with prior art luciferase based analyses. At least one reason for this is that it is not viable to isolate cells expressing a particular level of luciferase from comparable cells differing only in some feature of their luciferase expression.

### Selectable Markers

The nucleic acid of the present invention also comprises a selectable marker gene. A selectable marker gene allows cells carrying the gene to be specifically selected for or against, in the presence of a corresponding selection agent. Selectable markers can be positive, negative or bifunctional. Positive selection markers allow selection for cells carrying the marker, whereas negative selection markers allow cells carrying the marker to be selectively eliminated. A bifunctional selectable marker contains means for either positive or negative selection of cells containing the selectable marker gene or fusion gene (see Schwartz et al Proc. Natl. Acad. Sci USA 88:10416-10420 (1991)).

The use of selectable markers in the nucleic acids and techniques of the present invention leads to several advantages noted herein. One such advantage is it permits the selection of cells harbouring genetic constructs of interest. Furthermore, the use of multiple selectable markers can allow a more complex selection regime to be implemented. For example, by using two selectable markers a first population of cells can be selected harbouring nucleic acids of a library, and a second selectable marker may be used to select those cells which down regulate expression via the UTR following miRNA addition.

Typically, a selectable marker gene will confer resistance to a drug (e.g. prodrug convertase) or compensate for a metabolic or catabolic defect in the host cells. For example, selectable markers commonly used with mammalian cells include the genes for adenine deaminase (*ada*), hygromycin B phosphotransferase (*Hph*), dihydrofolate reductase (DHFR), thymidine kinase (TK), thimidylate kinase (which converts AZT and may be more powerful than thymidine kinase), glutamine synthetase (GS), asparagine synthetase, and genes encoding resistance to neomycin (G418), puromycin, histidinol, zeocin (zeocin may be substituted with bleomycin and/or thleomycin for which the resistance gene is the same for all three; zeomycin is typically suitable due to its lower cost) and Blasticidin S.

Selection agents are used according to manufacturer's recommendations where appropriate. As a guide, ZEO selection can take about 3 weeks, PURO selection can take about 1 week. Concentrations and conditions including level of expression of the selectable marker may all be manipulated by the skilled worker to vary the selection times according to need.

The selectable marker gene may be any gene which can complement a recognisable cellular deficiency. Thus, for example, the gene for HPRT could be used as the selectable marker gene sequence when employing cells lacking HPRT activity. Thus, this gene is an example of a gene whose expression product may be used to select mutant cells, or to "negatively select" for cells which express this gene product. Another example is use of the selectable marker gene puromycin N-acetyltransferase (Pac) which confers resistance to the drug puromycin on cells carrying the gene.

Another common selectable marker gene used in mammalian expression systems is thymidine kinase. Cells that do not contain an active thymidine kinase (TK) enzyme are unable to grow in medium containing thymidine but are able to grow in medium containing nucleoside analogs such as 5-bromodeoxyuridine, 6-thioguanine, 8-azapurine etc. Conversely, cells expressing active thymidine kinase are able to grow in media containing hypoxanthine, aminopterin, thymidine and glycine (HATG medium) but are unable to grow in medium containing nucleoside analogs such as 5-azacytindine (Giphart-Gassler, M et al Mutat. Res. 214:223-232 (1989), Sambrook et al, In: Molecular Cloning A Laboratory Manual, 2nd Ed, Cold Spring Harbour Laboratory Press, N.Y. (1989)). Cells containing an active Herpes Simplex Virus Thymidine Kinase gene (HSV-TK) as a selectable marker gene are incapable of growing in the presence of gangcylovir or similar agents. Clearly the agent used to implement the selection should be used according to the manufacturer's instructions. It may be that the concentration or mode/timing of addition of the agent to the cells might need to be optimised for the particular constructs or selectable markers used in order to provide the most robust and reliable selection. This optimisation is well within the abilities of the person skilled in the art. It may even be that a split-level selection strategy might be implemented, for example with enhanced levels of the agent of interest to select the highest expressing clones, or vice versa with a lower level to select lower expressing clones. Such variations are well within the ambit of the skilled person working the invention.

Moreover, mutants of metabolic enzymes have been created which allow for greater drug sensitivity. For instance thymidylate kinase F105Y increases the sensitivity of cells to AZT, which in turn may permit less AZT to be used, or may achieve a faster killing for a given concentration of AZT. R16GLL mutant may also be used. In addition, a mutant HSVTK named SC39 has been shown to be significantly more sensitive to gancyclovir and/or similar agents (Blumental et al, Mol. Therapy, 2007). Thus, mutants of known selectable markers also find application in embodiments set out herein.

Thus for negative selection HSVTK, Thymidylate kinase (such as F105Y or others) may be used. For positive selection, PURO, ZEO, HYGRO or even NEO may be used. Suitably fusions of the invention comprise one positive and one negative marker from these groups. Suitably the fusions may be in either order. Most preferred are those in the examples section. Indeed, these have been shown successfully to work as illustrated which may not be assumed from an understanding of their behaviour in other contexts.

Some fusions exist prior to the invention such as TK/ZEO (Cayla/Invitrogen) or HYGRO/TK (Immunex). These are known only for gene therapy type applications e.g. for killing cells which received the vector after treatment is concluded (i.e. use as suicide gene). Combinations or fusions disclosed herein for the first time are preferred. In any case, fusion to regulatory RNAs as taught by the invention has not been previously described or suggested.

Furthermore, selectable markers need not always involve cell killing e.g. green fluorescent protein (GFP)/PURO may be used (as other fluors or visualisable proteins) for flowsort selection i.e. flowsort selectable marker.

Particularly suitable combinations include TK/PURO, wtThym/PURO, R16GLLThym/PURO, F105YThym/PURO, R16GLL-F105YThym/PURO, F105YThym/Zeo, Zeo/F105YThym, GFP/PURO.

It is disclosed that it may be that a dual selectable strategy can be used with a single selectable marker. In this scenario, it would be necessary to choose the selectable marker in such a way that it affords both positive and negative selection. For example, the metabolic enzyme encoded by the URA gene can provide independence of uracil in certain eukaryotic systems. Thus, cells harbouring the URA gene may be positively selected using uracil free medium - only those cells harbouring the URA gene will be able to grow by making their own uracil. The very same gene is capable of converting the precursor 5-fluoro-orotic acid (5-FA) into a toxic metabolite. Thus, cells harbouring the uracil gene can be selected against by inclusion of 5-FA into the growth medium - those cells harbouring the URA gene will convert it into a toxic metabolite and will be removed by the selection procedure. Thus, in this scenario, a single selectable marker can in fact provide both positive and negative selection steps. However, most commonly, positive and negative selection steps will be provided by the provision of two or more selectable markers.

In a similar manner, cytosine deaminase may be used as a selectable marker. Normal mammalian cells do not contain cytosine deaminase. Cells expressing the cytosine deaminase gene metabolise the relatively nontoxic prodrug 5-fluorocytosine to the highly toxic 5-fluorouracil. Thus, cytosine deaminase may be used as a selectable marker thus permitting negative selection when treated with 5-fluorocytosine in different embodiments.

Suitably multiple selectable markers are provided as fusions in a single open reading frame on the nucleic acid of the invention.

Suitably at least two selectable markers are used. Suitably three selectable markers are used. Suitably four selectable markers are used, or even more.

Suitably two selectable markers are used, suitably those two selectable markers are fused. 'Fused' has its ordinary meaning in the art, i.e. it means that suitably the markers may be expressed from a single open reading frame which encodes a polypeptide having the amino acid sequence of each of said markers. Thus 'fused' means that suitably the two or more selectable markers are provided in a single polypeptide (or a single nucleic acid or transcript encoding a single polypeptide comprising said two or more selectable markers). In other words, the open reading frames for the markers are 'fused' at the nucleic acid level resulting in expression of a 'fusion protein' which comprises the amino acid sequences for each of the two (or more) markers which are said to be 'fused'. This advantageously allows a dual selection screening procedure to be followed, for example positive selection for presence of the genetic construct followed by negative selection against those cells which fail to down-regulate expression in the presence of the miRNA be tested.

Thus, suitably the nucleic acid(s) encoding the two or more selectable markers provided as a single 'fusion' polypeptide does not have any stop codon in between the parts of the open reading frame encoding the two selectable markers.

Suitably selectable marker fusions are selected from the combinations of TK/PURO, TK/HYGRO, or TK/ZEO. Selectable marker fusions listed may typically be reversed e.g. HYGRO/TK or TK/HYGRO may be equally effective and should each be understood to be embraced by reference to "HYGRO/TK" or "TK/HYGRO". In case of any further guidance being needed, suitably as a default the fusion is as written e.g. HYGRO/TK means Nterminus-HYGRO-TK-Cterminus unless the context indicates otherwise. Most suitably, a selectable marker is a TK/PURO fusion. This has the advantage that puromycin is very potent. This is possibly the best selectable marker. Puromycin blocks protein synthesis. This allows a pure population of transfected cells to be selected in approximately one week under laboratory conditions.

Hygromycin is also a very potent selectable marker. Hygromycin is comparable to puromycin in its potency.

Zeomycin is an intercalating agent. Zeomycin has a slower mode of action compared to puromycin or hygromycin. This may be advantageous in certain situations.

Thus, suitably the selectable marker is a fusion of the HSVTK and PURO proteins. Suitably said fusion comprises SEQ ID NO: 1, suitably said fusion consists of SEQ ID NO: 1.

Other prodrug convertases can be used instead of HSVTK, e.g. beta-glucosidase or others mentioned herein, paraticularly as mentioned above (selectable marker genes).

In a broad embodiment, other ways of selecting cells such as bead selection could be used for the presence or absence of markers such as LNGFR on the cell surface.

### Promoters

The nucleic acid of the present invention comprises a promoter operably linked to a coding sequence encoding, for example, a selectable marker gene. The term "operably linked" means that the components described are in a relationship permitting them to function in their intended manner. A promoter operably linked to a coding sequence is positioned in such a way that expression of the coding sequence is achieved in conditions under which the promoter is active.

The term "promoter" refers to a polynucleotide sequence that controls transcription of a gene or sequence to which it is operably linked. A promoter includes signals for RNA polymerase binding and transcription initiation. The term promoter is well-known in the art and encompasses polynucleotide sequences ranging in size and complexity from minimal promoters to promoters including upstream elements and enhancers.

A promoter is usually, but not necessarily, positioned upstream of the coding sequence, the expression of which it regulates. Furthermore, the regulatory elements comprising a promoter are usually positioned within 2 kb of start site of transcription of a gene.

One of ordinary skill in the art will understand that the selection of a particular useful promoter depends on the exact cell lines and other various parameters of the expression vector to be used to express the coding sequence. A large number of promoters including constitutive, inducible and repressible promoters from a variety of different sources are well known in the art and can be identified in databases such as GenBank and are available as or within cloned polynucleotides, from for example, depositories such as ATCC as well as other commercial or individual sources.

Promoters suitable for use in the nucleic acids of the present invention include those derived from mammalian, microbial, viral or insect genes. Commonly used mammalian cell promoter sequences are derived from polyoma virus, adenovirus, retroviruses, hepatitis-B virus, simian virus 40 (SV40) and cytomegalovirus. Minimal promoters such as the herpes simplex virus thymidine kinase promoter (HSVtk) may also be used. Mammalian promoters such as the beta actin promoter are also suitable for use in the nucleic acids of the present invention. Promoters from the host cell or a related species may also be suitable.

The constitutive cytomegalovirus immediate early promoter can be used to obtain a high level of gene expression in mammalian cells. Such promoters are widely available and can be obtained for example from Stratagene (for example the pCMV-Script® Vector). Another constitutive promoter, the SV40 enhancer/promoter (including the late or early SV40 promoter), is commonly used in the art and enables a moderately high level of gene expression in mammalian cells.

It may also be advantageous for the promoters to be inducible. With inducible promoters, the activity of the promoter increases or decreases in response to a signal. For example, the tetracycline (tet) promoter containing the tetracycline operator sequence (tetO) can be induced by a tetracycline-regulated transactivator protein (tTA). Binding of the tTA to the tetO is inhibited in the presence of tet. The Tet-On and Tet-Off Gene Expression Systems (Clontech) use a tetracycline responsive element to maintain recombinant protein expression in an on (constitutively off but induced with tetracycline) or off (constitutively on, but repressed with tetracycline or doxycycline) mode. Details of other suitable inducible promoters including jun, fos and metallothionein and heat shock promoters, may be found in Sambrook et al, In: Molecular Cloning A Laboratory Manual, 2nd Ed, Cold Spring Harbour Laboratory Press, N.Y. (1989) and Gossen et al Curr Opi Biotech 5:516-520 (1994).

In addition, any of these promoters may be modified by the addition of further regulatory sequences, for example enhancer sequences operably linked to the coding sequence. An enhancer is a cis-acting DNA element that acts on a promoter to increase transcription. An enhancer may be necessary to function in conjunction with the promoter to increase the level of expression obtained with a promoter alone. Operably linked enhancers can be located upstream, within or downstream of coding sequences and may be considerable distances from the promoter.

### Transcription terminator

The nucleic acids of the present invention may also comprise a transcription terminator. A "transcription terminator" refers to a nucleotide sequence normally represented at the 3' end of a gene of interest or the stretch of sequences to be transcribed that causes RNA polymerase to terminate transcription.

A separate genetic element is the polyadenylation signal, which facilitates the addition of polyadenylate sequences to the 3' -end of a primary transcript. The polyadenylation signal sequence includes the sequence AATAAA located at about 10 -30 nucleotides upstream from the site of cleavage, plus a downstream sequence. The polyadenylation signal may be located very near to the transcriptional terminator (when present) or may even overlap with it in some circumstances.

Generally, most transcriptional terminators include a GC rich sequence preceding the termination site and a sequence of T-residues in the non-template DNA strand attached to the termination site. The RNA polymerase traverses the GC-rich sequence to produce mRNA which can form a stable base-paired stem-and-loop structure within the mRNA. Transcription then usually terminates just downstream from the stem-and-loop structure where the T-residues result in a RNA ending with a sequence primarily comprising uridylate residues (Brennan and Geiduschek, 1983, Nucleic Acids Res. 11:4157).

An example of a terminator sequence is that from the bovine growth hormone gene. This terminator element may also provide the polyadenlyation signal. Terminator sequences may also be obtained from well known commercial suppliers such as the ZAP Express® Vector System (Stratagene) and the pCMV-V5-His6 (available from Clontech Laboratories (Palo Alto, Calif.). Terminators active in mammalian expression systems are described in the literature and easily obtained by the person skilled in the art.

### Transfection/Transduction

"Cell transfection" refers to the introduction of foreign nucleic acid into a cell. There are several methods of introducing DNA and RNA into a cell, including chemical transfection methods (liposome-mediated, non-liposomal lipids, dendrimers), physical delivery methods (electroporation, microinjection, heat shock), and viral-based gene transfer (retrovirus, adeno-associated virus, and lentivirus). The method of choice will usually depend on the cell type and cloning application and alternative methods are well known to those skilled in the art. Such methods are described in many standard laboratory manuals such as Davis et al, Basic Methods In Molecular Biology (1986).

Transfected genetic material can either be expressed in the cell transiently or permanently. In transient transfection, DNA is transferred and present in the cell, but nucleic acids do not integrate into the host cell chromosomes. Typically transient transfection results in high expression levels of introduced RNA 24-72 hours post-transfection, and DNA 48-96 hours post-transfection. Stable transfection is achieved by integration of DNA vector into chromosomal DNA and permanently expressed in the genome of the cell.

Transfection using commercially available liposomes such as Lipofectinamine 2000, electroporation or any other form of transduction can be used. Furthermore the nucleic acid such as the microRNA of interest can be cloned into viral or non-viral expression plasmids which can than be introduced by infection (viral vectors) or transfection (non-viral). This will result in stable transduction of the cells. Such details are common and well known to persons skilled in the art. In particular, such techniques may be practised as in Sambrook, E. F. Fritsch, and T. Maniatis, 1989, Molecular Cloning: A Laboratory Manual, Second Edition, Books 1-3, Cold Spring Harbor Laboratory Press; Ausubel, F. M. et al. (1995 and periodic supplements; Current Protocols in Molecular Biology, ch. 9, 13, and 16, John Wiley & Sons, New York, N.Y.).

Chemical means of transfecting cells with foreign nucleic acid include use of DEAE-dextran, calcium phosphate or artificial liposomes. DEAE-dextran is a cationic polymer that associates with negatively charged nucleic acids. An excess of positive charge, contributed by the polymer in the DNA/polymer complex allows the complex to come into closer association with the negatively charged cell membrane. It is thought that subsequent uptake of the complex by the cell is by endocytosis. This method is successful for delivery of nucleic acids into cells for transient expression. Other synthetic cationic polymers may be used for the transfer of nucleic acid into cells including polybrene, polyethyleneimine and dendrimers.

Transfection using a calcium phosphate co-precipitation method can be used for transient or stable transfection of a variety of cell types. This method involves mixing the nucleic acid to be transfected with calcium chloride, adding this in a controlled manner to a buffered saline/phosphate solution and allowing the mixture to incubate at room temperature. This step generates a precipitate that is dispersed onto the cultured cells. The precipitate including nucleic acid is taken up by the cells via endocytosis or phagocytosis. This has been accomplished on a large scale for mammalian cells for example as taught in J R Rayner and T J Gonda ("A simple and efficient procedure for generating stable expression libraries by cDNA cloning in a retroviral vector." Mol Cell Biol. 1994 February; 14(2): 880-887).

Transfection using artificial liposomes may be used to obtain transient or longer term expression of foreign nucleic acid in a host cell. This method may also be of use to transfect certain cell types that are intransigent to calcium phosphate or DEAE-dextran.

Liposomes are small membrane-bound bodies that can actually fuse with the cell membrane, releasing nucleic acid into the cell. A lipid with overall net positive charge at physiological pH is the most common synthetic lipid component of liposomes developed for transfection methods using artificial liposomes. Often the cationic lipid is mixed with a neutral lipid such as L-dioleoylphosphatidyl-ethanoloamine (DOPE). The cationic portion of the lipid molecule associates with the negatively charged nucleic acids, resulting in compaction of the nucleic acid in a liposome/nucleic acid complex. Following endocytosis, the complexes appear in the endosomes, and later in the nucleus. Transfection reagents using cationic lipids for the delivery of nucleic acids to mammalian cells are widely available and can be obtained for example from Promega (TransFast™ Transfection Reagent).

### Further Advantages

The use of a selectable marker in the study of miRNA function has not previously been disclosed. As noted above, analysis in this field has typically been confined to use of quantifiable markers such as luciferase. In trying to quantify the effects on protein expression of particular miRNAs, luciferase is particularly attractive. This allows directly comparable measurements of luminescence to be made and compared across different treatments. In sharp contrast, selectable markers operate on a more binary basis. The fundamental concept of a selectable marker is that cells harbouring the marker can be made to survive, and cells without the marker (or not expressing the marker) can be eliminated. Thus, the use of selectable markers in the field of miRNA analysis can be considered to be counter-intuitive. In addition, compared with the prior art use of luciferase, the use of selectable markers represents a loss of information. This is because, as noted above, luciferase is very well adapted for quantification and for comparison of expression levels between treatments, which information is rarely available or measured using selectable markers. Thus, the methods and materials of the present invention can be considered to be counter-intuitive with regard to the prior art. Clearly, in a field such as miRNA analysis, which is so closely based on comparative expression levels, the idea of converting to a system permitting only binary analysis from the background of a system which permits wide ranging direct proportional measurements and inferences regarding protein expression to be made would be dismissed out of hand. *A priori,* this would certainly appear to be a step backwards in terms of the information which can be usefully extracted out of such an analysis. However, as demonstrated herein, it is in fact surprisingly useful to employ genetic selection techniques in the analysis of miRNA function, and particularly to the identification of targets of said miRNAs.

It is an advantage of the invention that a directional cloning strategy is used. In a preferred embodiment, SfiI cloning is used. This is a rare cutting restriction enzyme. SfiI cuts at an 8 base pair recognition sequence. Furthermore, SfiI cuts leaving an a symmetric overhang at the two cut ends. This advantageously permits directional cloning strategies following SfiI digestion. These techniques are disclosed in the prior art such as in US Patent No. 5,595,895, which is incorporated herein by reference. Clearly, the invention embraces any directional cloning system suitable for use in a nucleic acid construct such as BstXI cloning. The restriction enzyme(s) used for directional cloning may be BstXI. This is also described in US patent number 5,595,895. SfiI directional cloning is preferred due to its simplicity. A further advantage of using SfiI cloning is that an 8 base pair recognition sequence is relatively rare in the genome. For example, if more frequent cutting restriction enzymes such as Spe I or Hind III are used, then there is a correspondingly greater risk of them digesting the target sequences during the cloning operation, which risk is reduced with the use of a longer recognition sequence such as an 8 base pair recognition sequence.

In contrast to expression vectors in other fields, it is preferred that nucleic acids of the present invention feature stop codons following the selectable marker. In this embodiment, when the selectable marker is a polypeptide encoded by the nucleic acid, translation of said selectable marker polypeptide is terminated at the stop codon. Thus, whether or not any sequence present in the nucleic acid of the invention as a 3 prime UTR or a candidate for 3 prime UTR encodes any polypeptide should not affect the operation of the invention. Indeed, it may be an advantage of the invention that any such coding sequence present in the 3 prime UTR or candidate 3 prime UTR will ideally not be fused to the polypeptide of the selectable marker. Thus, the stop codon or stop codons (suitably a stop box) present immediately after the open reading frame encoding the selectable marker polypeptide has the advantage of preventing or at least discouraging translation of any further downstream nucleic acid sequences.

A stop box is a genetic element commonly known in the art. In summary, a stop box comprises at least three stop codons, which are arranged in either an overlapping or a non overlapping format such that between the 5 prime end and the 3 prime end of the stop box a stop codon is presented in each of the three possible forward reading frames. The stop codons may overlap, or they may be separated by a small number of nucleotides, such as separated by one, two, four, five or more nucleotides. Clearly, the stop codons are unlikely to be separated by three, six, nine or any other number of nucleotides divisible by three since stop codons arranged in this manner would not be presented in different reading frames. However, it should of course be noted that two or more stop codons in frame are also useful, for example to guard against read-through, and may thus be employed in suitable embodiments, for example using repeated or duplicated stop codons, or even stop boxes, as appropriate. Such details are well known to a person skilled in the art.

### cDNA Libraries

Suitably the 3 prime UTR's or candidate 3 prime UTR's are derived from cDNA libraries. Suitably the cDNA's are mammalian cDNA's. Suitably the cDNA's are from a tissue or disease of interest. For example, the cDNA's may be from brain. This has the advantage of being a tissue presenting the most diverse cDNA's. In this way, cDNA's may be prepared from a single tissue but have the maximum chance of representing the greatest possible number of different genes. In another embodiment, cDNA's may be from a disease of interest. An example of such a disease is acute myeloid leukaemia. In this embodiment, suitably the cDNA's are all derived from acute myeloid leukaemia cells. This has the advantage of presenting 3 prime UTR's or candidate 3 prime UTR's which are likely to be of relevance to the chosen disease.

In principle, the 3 prime UTR's or candidate 3 prime UTR's may be derived from any suitable genetic source. cDNA libraries are a particularly convenient source from which to access 3 prime UTR's of candidate 3 prime UTR's. Using cDNA's as the source for the UTR's of interest has several advantages. Firstly, cDNA libraries may be oligo-dT selected, for example alone or in combination with random hexamers. This has the effect of making the libraries the most robust at the 3 prime end, which end adjoins the poly A tail. Due to their method of preparation, cDNA libraries have a tendency to be under-represented at the 5 prime end, particularly for the longest cDNA transcripts. However, this will have a minimal effect (if any) on the use of cDNA library as a source of 3 prime UTR's or candidate 3 prime UTR's since the 3 prime end of cDNA libraries is typically the best represented with the most intact and diverse sequences.

Of course, there may be miRNA target sites also present within the 5 prime UTR of genes or at other locations. Therefore, the use of a combination of oligo-dT and random hexamers advantageously allows a greater coverage of candidate miRNA target sites by a cDNA library so produced.

Since cDNA libraries are traditionally used for the study of the encoded polypeptides, it is itself surprising that such materials can be used as a source of diverse UTR's or candidate UTR's.

Optionally the candidate 3' UTRs can be size-selected. This has the advantage of optimising the size of the overall nucleic acid. This has the further advantage of allowing optimisation of the chances of including the greatest possible number of intact 3' UTRs based on knowledge of the most common sizes of 3' UTRs in the organism or tissue of interest from which the 3' UTRs are derived.

### Host Cells

The assays of the invention are advantageously carried out in (or on) host cells. Suitably these are eukaryotic cells. Suitably these are cells from a multicellular organism. Suitably the cells are from insects or vertebrates. When the cells are from vertebrates, suitably they are mammalian cells. Suitably the cells are 'cognate' to the miRNA or 3' UTR being studied, suitably the cells are cognate to both the miRNA and 3' UTR being studied. Being cognate preferably means derived from the same organism. This has the advantage that cellular processing machinery, for example for processing the miRNAs or for translating the mRNAs, will be common and will therefore provide the biologically most relevant conditions for studying or testing the miRNA-3' UTR function.

In some embodiments, it is desirable for the host cells to be different from the source of the miRNA and/or 3' UTR being studied. One example of such an application is when there are endogenous miRNAs which might interfere with or interact with the target sequence (e.g. 3' UTR or candidate 3' UTR) under study. In this embodiment it may be desirable to use cells or cell lines which are from a different organism to the organism(s) from which the miRNA and/or target sequence is derived. For example, when studying human miRNAs it may be desirable to use insect cells such as Sf9 cells. In this manner, it may be possible to avoid 'interference' or complication of the study or screen by naturally occurring or endogenous miRNAs. It is of course straightforward to test whether or not there are endogenous interfering miRNAs in cells or cell lines of interest by introducing nucleic acid bearing the target sequence(s) into the cell or cells and testing for expression of the selectable marker(s). If no expression is seen even in the absence of addition or introduction of miRNAs of interest, then it may be an indication that naturally occurring or endogenous miRNAs are preventing or downregulating expression of the selectable markers. Such an observation is an indication that this problem needs to be addressed before meaningful study or screen is undertaken, for example by testing an alternate cell or cell line until conditions for reliable expression of the selectable marker gene(s) in the absence of exogenous miRNA are established. This is clearly a routine matter for the skilled operator given the guidance provided herein.

Suitably the host cells contain at least the necessary apparatus for miRNA processing and for protein expression. Again, this is easily tested by introducing nucleic acid(s) of the invention and monitoring marker gene expression as noted above.

Suitable cells include 3T3 cells such as NIH 3T3 mouse fibroblasts (although these cells express MIR10a and MIR130); human HL60 or Jurkat cells (which advantageously do not express significant MIR10a or MIR130); human HeLa cells (which advantageously express very low MIR10a and MIR130); Cos cells (which are advantageously easily transfectable).

NIH3T3 and HeLa cells have the additional advantage of being easily transfectable.

Most suitably the cells are MCF7 cells.

In library or screening format, cell lines can be regarded as 'self cleaning' in the sense that UTRs won't get past the first round of screening/selection if their miRNA is expressed endogenously in the host cells used.

Particularly suitable are cells or cell lines as indicated in the examples section.

### Further Applications

MicroRNA's play a role in many biological processes such as differentiation, angiogenesis, cell adhesion, cell proliferation, survival and play a important role in haematopoiesis. They have also been shown to play important roles in cancer. Therefore the invention can advantageously be applied in many different areas of industry.

We describe a functional assay developed for the identification of microRNA targets which can identify multiple targets for a specific micro RNA in one procedure. This finds application across the expanding field of microRNA study.

Adaptation of the selection procedure can advantageously make this invention usable in connection with miRNAand/or miRNA targets from diverse organisms. Moreover, the identification of microRNA targets is important in diseases such as cancer where microRNA's play important roles. The identified targets may provide novel targets for small molecule development (e.g. BCR/ABL, glivec, and others).

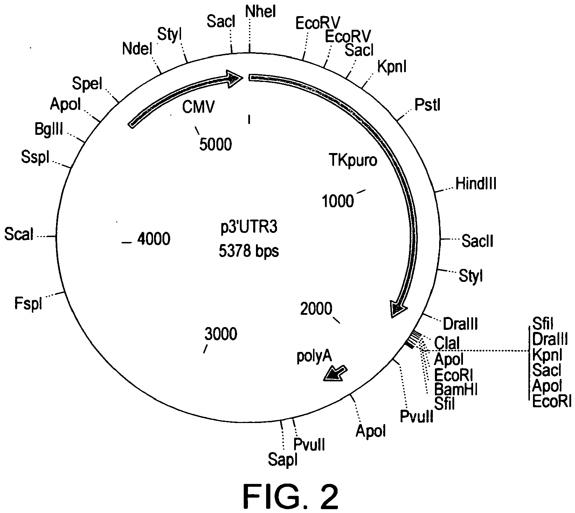

In addition, the invention provides new plasmid(s) for cloning UTR's behind HSVTK/puro, for example as shown in Figure 2.

The invention also provides novel selectable marker fusion(s).

There may be miRNA target sites also present within the 5 prime UTR of genes. Therefore, the use of a combination of oligo-dT and random hexamers may advantageously allow for a greater coverage of target sites by the cDNA library as compared to use of oligo-dT alone.

### Regulators of regulatory RNA

Using a target for a specific microRNA, the system can be used to identify regulators of this microRNA. A population of cells expressing the target sequence (e.g. target UTR) linked to selectable markers (such as the TKpuro fusion) and microRNA will be gancyclovir resistant and puromycin sensitive. If a substance or cDNA library is then introduced to these cells and selected in puromycin we can identify genes which regulate this microRNA expression, i.e. genes or substances which prevent or inhibit the miRNA action and therefore permit or increase selectable marker expression (which is repressed in the absence of the gene or substance).

In other words, this system can be used to study or screen for genes, chemicals, small molecules or other entities which regulate regulatory RNA such as microRNA. For example, if mirX regulates target Y, then to identify entities or treatments that down-regulate mirX expression, the substance or gene (e.g. a cDNA library or small molecule library) would be introduced to the cells. Down-regulation of mirX would result in expression of selectable marker such as puroTK and confer puromycin resistance onto these cells. When the test entitiy is cDNA, identification of the introduced cDNA will reveal gene(s) that regulate mirX expression and/or function. When the entity is a small molecule, such small molecule libraries may be advantageously applied in single experiments or pools of multiple compounds as is well known in the art and often advantageously automated e.g. by use of robotic sample handling.

### Off-Target Screening

Many regulatory RNAs, such as siRNA molecules, are under development for or in clinical trials. Embodiments of the invention can be used to screen these siRNA molecules for off target effects of the siRNA. This is an important additional industrial application and utility of this system.

In these embodiments, the system can be used to study off-target effects of regulatory RNA such as small interfering RNA (siRNA). Many siRNA molecules are under development in clinical trials for knockdown of genes such as oncogenes (e.g. BCL2) in cancer and/or mutant genes involved in other genetic diseases. A problem with individual siRNAs is off target effects due to the seed sequence (hexamer sequence at 5' end of siRNA or microRNA). It is impractical to design siRNA without a seed sequence that, except from the intended target, is absent in the human genome. This is simply due to the size of the human genome and the probability of such a short sequence (e.g. a 6mer) being unique in the genome. This seed sequence would be expected to occur hundreds of times in the human genome. siRNA with off target seed sequence(s) could act as a microRNA (only partial homology with the target instead of 100% homology as for siRNA) at these inappropriate or off-target sites. The system described herein could be used to test proposed siRNA molecules for possible off target effects. Suitably full length cDNA libraries could be used as a source of candidate regulatory RNA target sequences in nucleic acids of the invention. This has the advantage of being more likely to cover all possible seed sequences as compared to truncated cDNAs or other sources, althougth of course those could equally be used if desired.

The invention is now described by way of example. These examples are intended to be illustrative, and are not intended to limit the appended claims.

### Brief Description of the Figures

Figure 1 shows a diagram of method(s) of the invention.
Figure 2 shows a diagram of a nucleic acid of the invention.

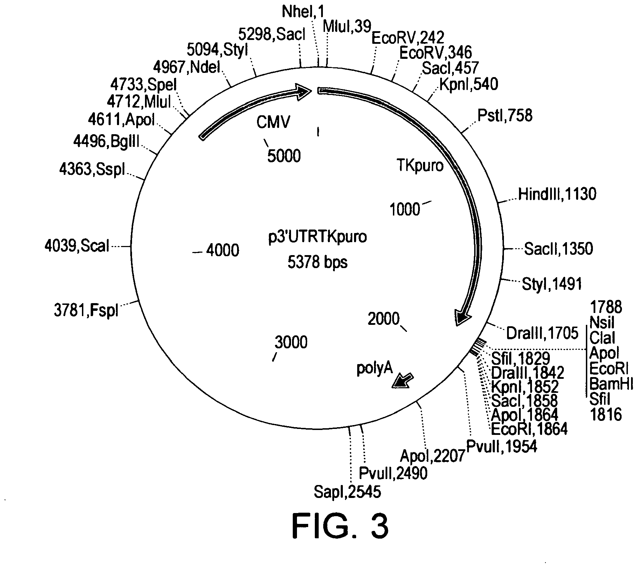

Figure 3 shows a diagram of a nucleic acid of the invention.

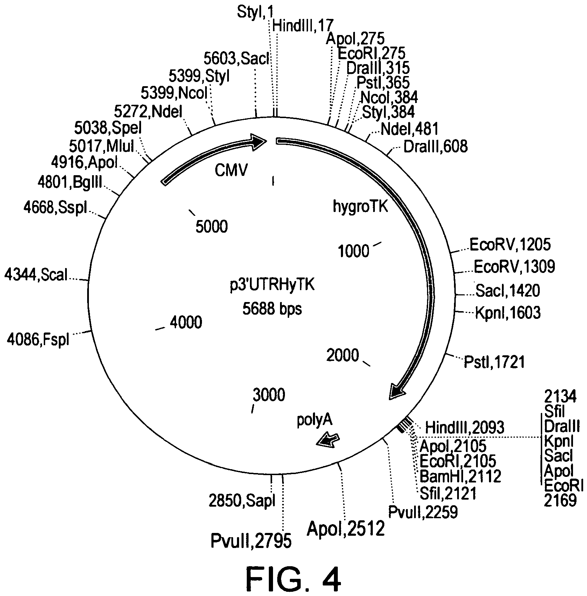

Figure 4 shows a diagram of a nucleic acid of the invention.

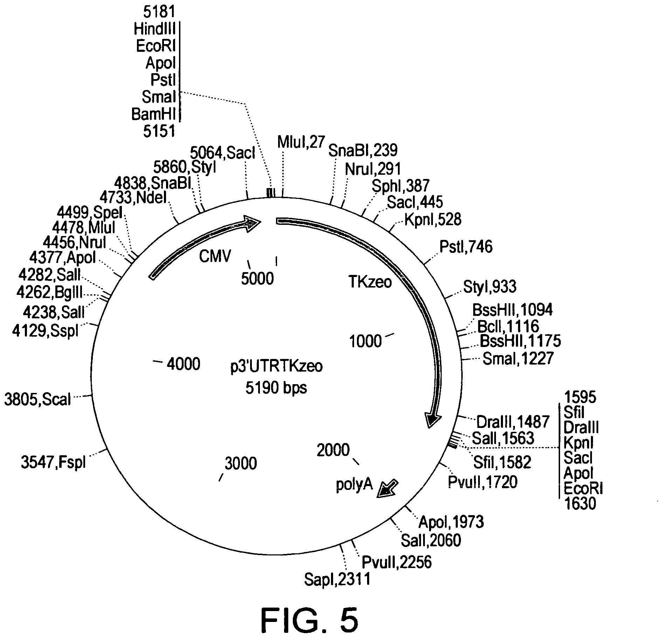

Figure 5 shows a diagram of a nucleic acid of the invention.
Figure 6 shows a bar chart of Luciferase/MAFB UTR down regulation of expression and a photograph of MAFB protein expression.
Figures 7 and 8 show bar charts of GCV Sensitivity Day 10.
Figure 9 shows a bar chart of mir-10a mir-130a Expression.
Figure 10 shows a bar chart of TKZEO Gancyclovir 7d.
Figure 11 shows a bar chart of TKZEO Ganciclovir 13d.
Figure 12 shows a bar chart of AZT sensitivity Day 7.
Figure 13 shows Mir10a and mir130a Expression from MCF7 cells transient (upper) and stable (lower)
Figure 14 shows a photograph of representative brain UTR library of the invention.
Figure 15A shows size selected cDNA; figure 15B shows cloned library Sfi I digested.
Figure 16 shows PCR analysis of library.

### Examples

### Example 1: Nucleic Acids

A nucleic acid is constructed comprising the following contiguous elements arranged in the 5 prime to 3 prime direction; a promoter; a selectable marker; a cloning site for receipt of a nucleic acid segment, said segment comprising a candidate miRNA target sequence; and a poly adenylation signal.

The elements are arranged such that a transcript directed by said promoter comprises said selectable marker, said candidate miRNA target sequence, and said poly adenylation signal in that order.

### Example 2: Dual Selectable Markers

As explained herein, the selectable marker is a key part of the present invention. In certain embodiments, the selectable marker may advantageously comprise more than one activity. This example demonstrates the production of selectable markers with more than one activity. In this example, this is accomplished by fusion of the ORFs for two different individual selectable markers into a single nucleic acid segment. This advantageously results in the production of a single polypeptide comprising two different polypeptide domains, each having its specific (selectable) activity.

In this example, the two individual markers used are HSVTK and PURO. These are fused to form a TK/PURO dual selectable marker.

The open reading frames of HSVTK and PURO are studied. A suitable fusion point is selected with consideration to the nature of the polypeptide products in order to maximise the chances of their activity being retained in the fused product. At this stage, a decision can be taken whether or not to include a linker (e.g. a linker region or a 'tether' or other such junction) at the join between the two polypeptides. Attention is also paid to practical matters such as scanning the nucleic acid sequences for restriction enzyme recognition site(s) which might interfere with the procedure or with use of the fusion in the invention (e.g. SfiI, BstXI, or other restriction enzyme sites intended to be used for UTR insertion in the eventual nucleic acid of the invention should advantageously be eliminated at this stage). Elimination of such sites may be suitably accomplished by site directed mutagenesis or similar technique.

The nucleic acid sequences are then produced and joined as necessary. This can be by any suitable means known in the art. For example, this may be by restriction enzyme digestion and ligation of the different elements together to form the fusion (including selective filling in or blunt-ending of any intermediate fragments as required). Alternatively this may be accomplished by PCR amplification of the desired fragments followed by cloning/ligation as appropriate. Alternatively the complete nucleic acid sequence designed may be directly synthesised in complete form, for example by chemical synthesis.

In this example, a Hygro/TK fusion is produced. This fusion has the sequence shown in SEQ ID NO: 3.

### Example 3: HSVTK/PURO dual selectable marker

In this example, the two selectable markers are fused to produce a single translation product comprising both activities/polypeptides.

In this example, the two individual markers used are HSVTK and PURO. These are fused to form a TK/PURO dual selectable marker.

The open reading frames of HSVTK and PURO are studied. The markers are then fused as described in example 2.

The resulting selectable marker is shown in SEQ ID NO: 1. This is a dual selectable marker. This is a TK-PURO fusion according to the present invention.

### Example 4: Nucleic Acid with dual selectable markers

In this example, two selectable markers are incoporated into the nucleic acid of the invention.

In this example, a nucleic acid with HSVTK/puro as selectable marker is produced.

The two selectable markers are fused to produce a single translation product comprising both activities/polypeptides as in example 3.

This nucleotide sequence encoding the dual selectable marker is then introduced into the nucleic acid of the invention after (i.e. downstream or 3' of) the promoter and before (i.e. upstream or 5' of) the site for 3' UTR insertion.

### Example 5: 3' UTR libraries

3' UTR libraries are produced according to the present invention.

A 3 prime UTR library is made by providing a nucleic acid as described above, such as described in example 1, and inserting into said cloning site a nucleic acid comprising a candidate miRNA target sequence. In this example the candidate miRNA target sequence is a 3 prime UTR or a candidate 3 prime UTR.

In more detail, the nucleic acid into which the 3'UTRs or candidate 3'UTRs is inserted is comprised by the nucleic acid of example 4. Specifically, the nucleic acid is comprised by plasmid p3' UTR3 (see figure 2).

In this example, the nucleic acid segments bearing the 3' UTRs or candidate 3' UTRs are or are derived from cDNAs. In this specific example, the cDNAs are derived from brain. Brain has the largest number of unique transcripts compared to any other organ. This advantageously allows creation of libraries with maximised diversity. Clearly, cDNAs from any tissue can be used, or indeed a mixture of cDNAs from different tissues can be used in order to maximise diversity.

We use an oligo-dT primed human brain cDNA library (as noted above, brain expresses the highest number of different mRNA's). In this cDNA library, the cDNA's have been directionally cloned into two SfiI. sites with different 3' overhangs (GGCCNNNNNGGCC).

On average, a human 3' UTR is ~1000 nt long. Therefore, the library is digested with SfiI and optionally size-selected i.e. the fraction below 1500bp is isolated to ensure capture of the majority of 3'UTRs. This cDNA is then directionally cloned into the SfiI site of the p3' UTR vector downstream of TKpuro.

Thus, a 3' UTR library according to the present invention is produced.

### Example 6: AML Libraries

The technique of example 5 is applied to the construction of a disease-specific 3' UTR library.

The 3' UTR's (candidate 3' UTR's) are derived from a cDNA library. In this example, that library is derived from acute myeloid leukaemia cells.

The cDNAs are optionally size-selected. In this example, they are size-selected with a maximum size of approximately 1500nt.

This cDNA is then directionally cloned into the SfiI site of the p3' UTR vector downstream of TKpuro.

Thus, a 3' UTR library according to the present invention is produced.

### Example 7: Cell based libraries

A plasmid library is produced according to example 5 or example 6 above and introduced at large scale into host cells. In this example, the cells are non-human cells and the introduction of the library into the cells is performed as described in Mourtada et al 2005 (Mourtada-Maarabouni M, Kirkham L, Farzaneh F, Williams GT. Functional expression cloning reveals a central role for the receptor for activated protein kinase C 1 (RACK1) in T cell apoptosis. J Leukoc Biol. 2005.2:503).

The cells containing the plasmid library are then selected in the presence of puromycin so that only cells which have taken up plasmid library can grow.

The cells are then expanded whilst preserving the diversity of the collection. The expanded cells are then pooled. Aliquots of the pooled expanded cells are then preserved for future use, for example by freezing and storage at -196°C in liquid nitrogen.

When required, cells are thawed and returned to culture for use in screening/analysis. Puromycin selection may be applied at any time to ensure that only cells harbouring the target plasmid are maintained. A collection of cells comprising the plasmid library in this manner is regarded as a cell based library according to the present invention.

### Example 8: Screening

The invention provides tools and methods for target identification and validation in miRNA gene regulation. Also provided are functional assays for the identification of miRNA targets, for example by library screening.

### Selection Study (Screening Study)

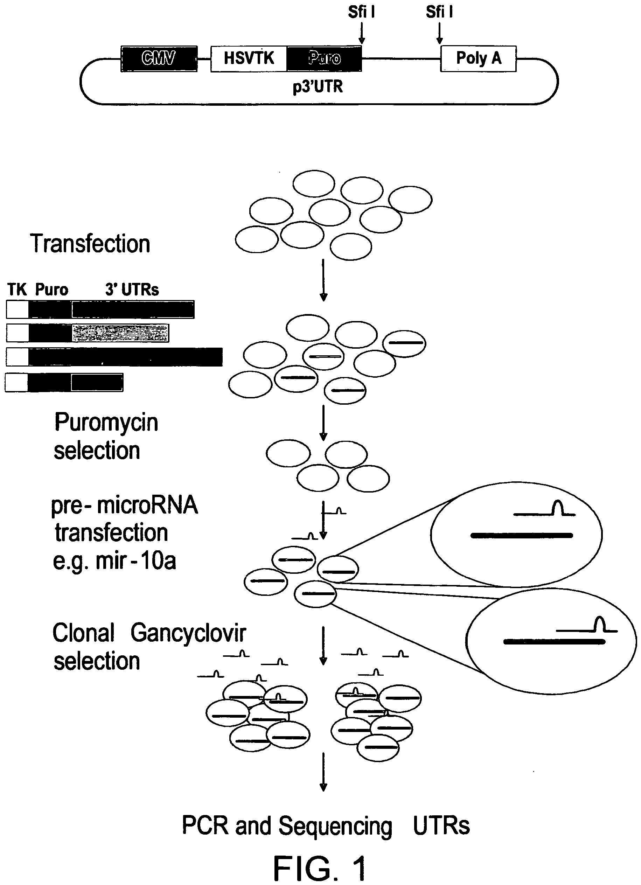

In this example, we apply a novel selection approach for the identification of protein downregulation due to miRNA binding to 3' UTRs. To this end we utilise 3' UTRs cloned downstream of a HSVTK/Puro fusion gene which, when expressed, confers puromycin resistance and gancyclovir sensitivity to cells. Downregulation of translation due to miRNA binding to the 3' UTR converts these cells to puromycin sensitivity and gancyclovir resistance (see Fig.1 for overview).

In order to demonstrate this approach, we cloned validated miRNA targets sites and the full-length 3' UTRs for HOXA 1 and MAFB genes downstream of TKpuro into the SfiI sites of p3' UTR (see Fig.2). HOXA1 and MAFB have known interaction with miRNAs mir-10a and mir-130a respectively (Garzon R, Pichiorri F, Palumbo T, et al. MicroRNA fingerprints during human megakaryocytopoiesis. PNAS 2006;103:5078-5083).

Murine or insect cells are transfected with the p3' UTR expression plasmids and selected in puromycin to obtain a population of transfected cells.

Precursor miRNA (mir-10a, mir-130a; Ambion) and scrambled control RNA oligo's are then transfected and the cells expanded in the presence of gancyclovir to isolate clones in which the miRNA has downregulated the TKpuro protein expression converting these cells to gancyclovir resistance.

Surviving cells are cloned and the presence of the HOXA1 and MAFB target sites or UTR's verified by PCR and sequencing.

Expression levels of TKpuro in the presence of the miRNAs may be investigated by western blotting for HSVTK using commercially available antibodies (Insight Biotechnology).

### Library Screening

A plasmid library is produced according to example 5 above and introduced at large scale into host cells. In this example, the cells are non-human cells and the introduction of the library into the cells is performed as described in Mourtada et al 2005 (Mourtada-Maarabouni M, Kirkham L, Farzaneh F, Williams GT. Functional expression cloning reveals a central role for the receptor for activated protein kinase C 1 (RACK1) in T cell apoptosis. J Leukoc Biol. 2005.2:503).

Following puromycin selection the miRNA of interest and/or control(s) is/are introduced. In this example, mir-10a, mir-130a or scrambled oligos are introduced.

Transfection using commercially available liposomes such as Lipofectinamine 2000, electroporation or any other form of transduction is used.

We then grow the library containing cells in the presence of gancyclovir and test resistant clones for the presence of the HOXA1 or MAFB 3' UTR in these clones.

This procedure also identifies a number of other targets for mir-10a and mir-130a. These are verified by western blot analysis of the TK/puro expression in these clones. This library screening technique is thus shown to be an invaluable tool for the identification and target validation for both known and as yet unidentified miRNA's.

### Example 9: Off-Target Screening

In this example, siRNA to knockdown a gene involved in liver cancer is the regulatory RNA of interest. Suitably this can be targeted specifically to the liver *in vivo.*

To investigate off target effects of this regulatory RNA, a brain or liver 3'UTR library or cDNA library coupled to selectable marker such as TKpuro would be tested as described above.

The siRNA under investigation is introduced to the cells.

Candidate target sequences from ganciclovir resistant colonies are then PCR'd and sequenced. If genes other than the intented target gene X are recovered then this is indicative of off-target effects of the regulatory RNA. These can then be assessed or further studied as appropriate.

These results aid the decision to proceed with or to design a different regulatory RNA such as siRNA.

### Example 10: Illustrative Library Screening

A) We have transfected MCF7 and MCF7mir130A with a UTR library spiked with 20% of MAFBUTR. They are selected in zeocin and all the controls are dead and many colonies are obtained. mir130A is introduced into the transfected MCF7 cells and then selected in puromycin (7-10 days) and than selected in gancyclovir. Clones are then sequenced.
B) In addition, 2 transfections were made into MCF7 and MCF7mir130A which do express mir130A. Because MCF7 do not naturally express mir130A after zeocin selection the clones recovered should contain a MAFBUTR in ~20% of the clones. However in MCF7mir130A the MAFBUTR should be silenced which results in the loss of zeocin resistance. The clones recovered after zeocin selection from this second transfection into MCF7mir130A should have no or very little MAFBUTR inserts.

DNA is then isolated from a mixed population of cells from both transfections and PCR the UTR inserts (mixed population). These inserts are cloned into the TA cloning vector and individual clones are sent for sequencing in 96 well format. Approximately 48 clones from each transfection are seqeunced.

We then count how often the MAFBUTR is present in clones from the MCF7 and MCF7mir130A transfection. Thus the principle of the procedure is demonstrated.

At the same time the procedure can be followed with GCV selection as well.

### Example 11: Further Library Screening

We have transfected MCF7 cells and MCF7(mir130) cells. MCF7 does not express mir130 and in MCF7(mir130) we have introduced mir130 and we have verified expression of mir130 by qPCR.

In a small scale experiment (10 plates of each) we have introduced a library which was cloned in the p3'TKzeo vector. The library was spiked with 20% MAFB UTR which is a target for mir130.

Both cell lines were selected in 1mg/ml zeocin which resulted in 200-300 colonies for each cell line. Because of the absence of mir130 in MCF7 the MAFB UTR should not be downregulated. Downregulation of the MAFBUTR should result in the absence of TKzeo protein which should result in the death of these cells in zeocin. In MCF7(mir130) the MAFBUTR should be downregulated which should result in the death of cells containing the MAFBUTR.

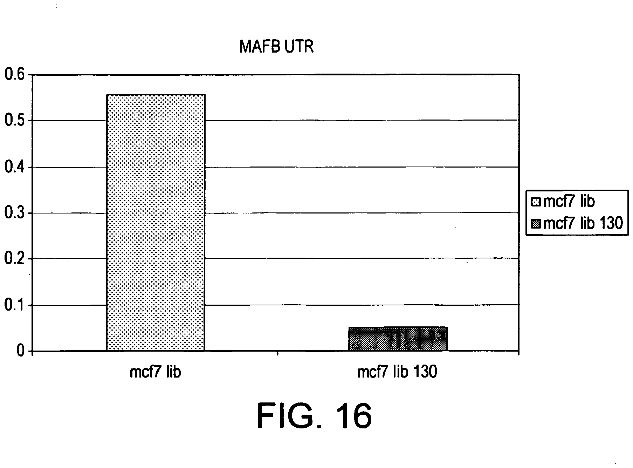

In conclusion; in MCF7 cells after selection in zeocin ~ 20% of clones should contain the MAFBUTR whilst in MCF7(mir130) the percentage should be much lower. To investigate this we designed primers that would only amplify the MAFBUTR DNA present in the library and not the endogenous MAFB. The results are presented in figure 16. There is a ~10X difference in the amount of MAFBUTR DNA between the two different cells which is a clear indication of the validity of the procedure.

We also PCR amplified the complete UTR's present in the two cells and cloned these PCR products in plasmids. 24 clones from each cell are sent for sequencing. There is still a 4 fold reduction in the number of MAFB containing clones.
Explanatory note: This may be an underestimate. Without wishing to be bound by theory, it may be that the plasmid preps are not equally clean. The MAFBUTR used for spiking was a maxiprep™ from Sigma™ and the library prep was a gigaprep™ from qiagen™. The sigma™ prep may be cleaner resulting in more transfected cells. Clearly this may be optimised by the skilled worker by cleaning the library prep according to any suitable technique known in the art.

Furthermore, we have now introduced mir 10, mir 130 and a short hairpin RNA (shRNA) against MAFButr into the MCF7 cells containing the library. These cells will be put under zeocin selection which should remove the MAFBUTR from the cells expressing mir130 or the shRNA but not from cells expressing mir10. In a separate experiment these cells may be put under Gancyclovir selection which should rescue the MAFBUTR from the cells expressing mir130 or the shRNA but not from cells expressing mir10.

### Example 12: Functional Assay

Selection is more powerful than conventional screening (where non-hits remain present rather than being lost or selected out); thus we employed a selection based screen as follows:
Drug Selection
Positive/Negative selection
Fusion protein of a selectable marker (e.g. puro, hygro, zeo or other suitable) with a prodrug convertase (e.g. HSVtk - GCV, Cytosine deaminase - 5FC, thymidylate kinase - AZT or other suitable)
GFP-puro fusion for screening and FACS sorting

### Example 13: 3' UTR library

A library is constructed according to the following:
Median length of 3'UTR is 1kB
Starting material: Brain cDNA library
Oligo dT primed: most inserts will contain at least partial 3'UTR
Directionally cloned using different Sfi I sites
Size selected >2.5 kB

### Example 14: Screening

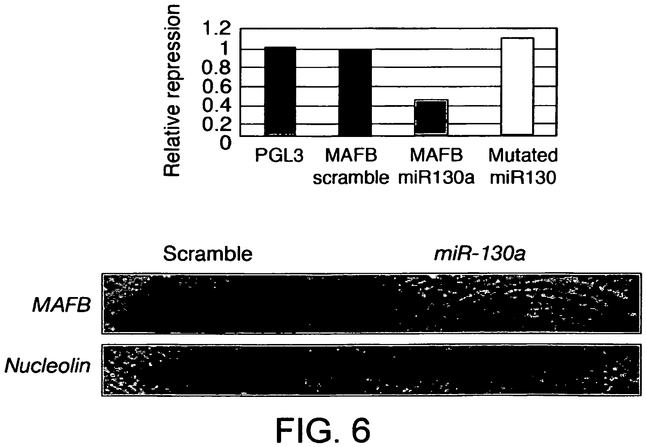

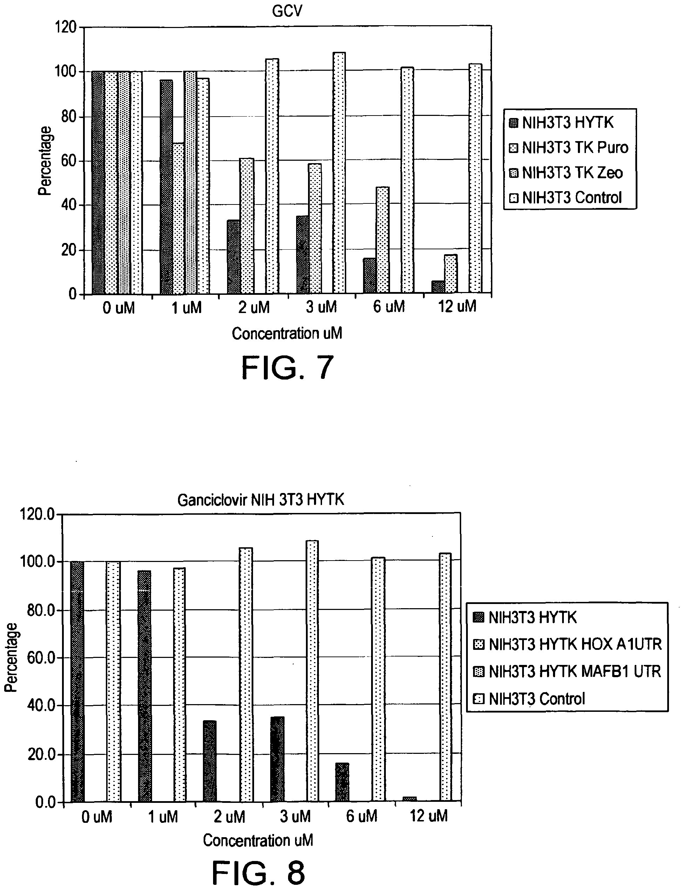

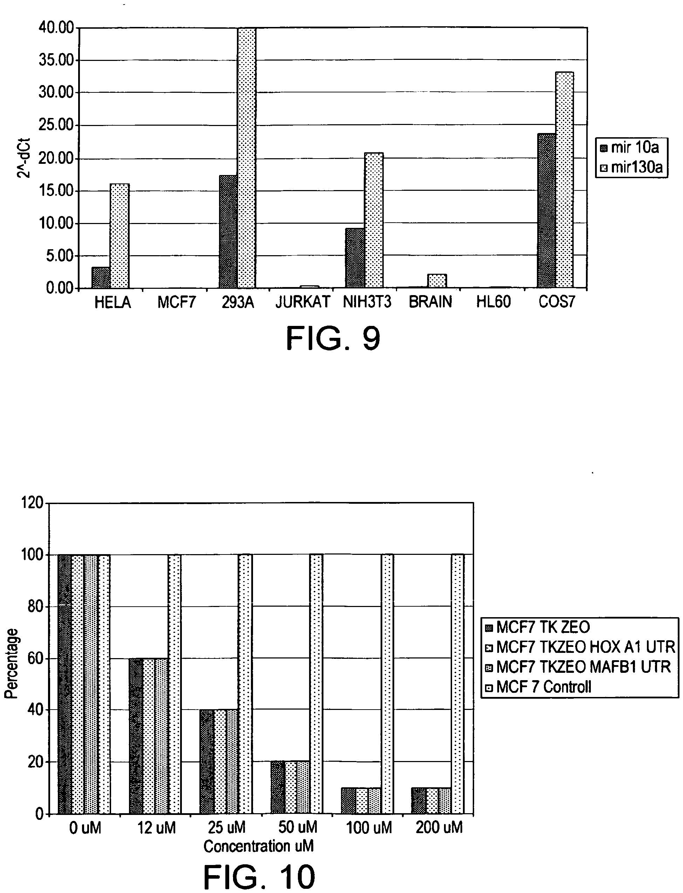

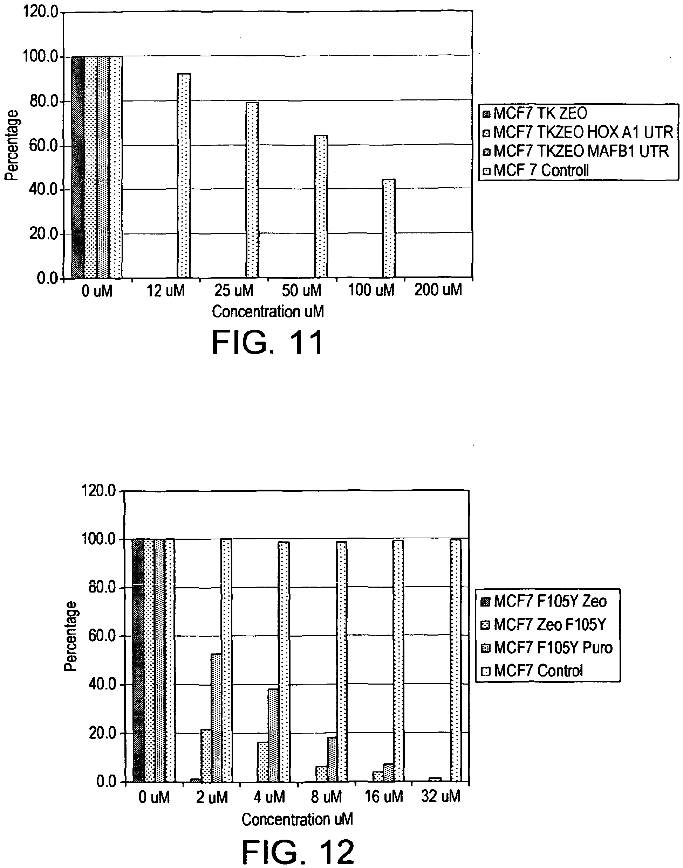

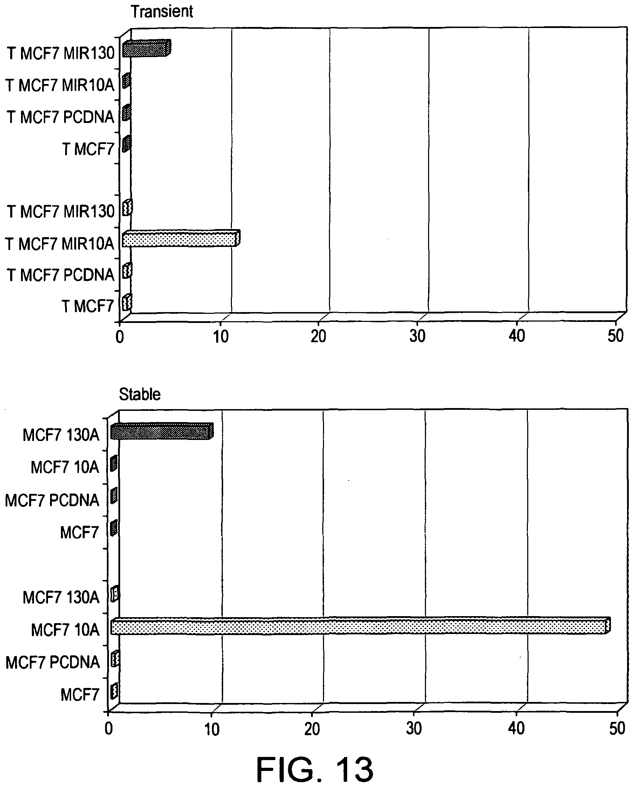

The HoxA1 and MAFB are down-regulated by mir10a and mir130 respectively.
HoxA1 and MAFB UTR's and predicted target sites cloned into pos/neg selection vector and a luciferase vector.
MAFB is a target of miR-130a (see Figure 6); Down-regulation of HOXA1 by mir10a has also been established.
GCV Sensitivity Day 10 is shown in Figures 7 and 8.
mir-10a mir-130a Expression is shown in figure 9.
TKZEO Gancyclovir `7d' is shown in figure 10, and '13d' in figure 11.
AZT sensitivity Day 7 is shown in figure 12.
Mir10a and mir130a Expression from MCF7 cells transient (upper) and stable (lower) is shown in figure 13.

### Example 15: Detailed Manufacture of Library

Library is manufactured as follows:
Size selected Sfi I digested cDNA >2.5Kb
Cloned in TKzeo Sfi I
15 µl Ligation 1µg TKzeo + 200ng LibraryTransformed 1.0 µl
Plated 1 µl and 10 µl out of 1000 µl
>500 colonies from 1 µl
500 x 1000 x 15 = 7.5 million
50 minipreps 50 different inserts
Collected ± 600.000 independent clones
7.5 mg from Giga prep

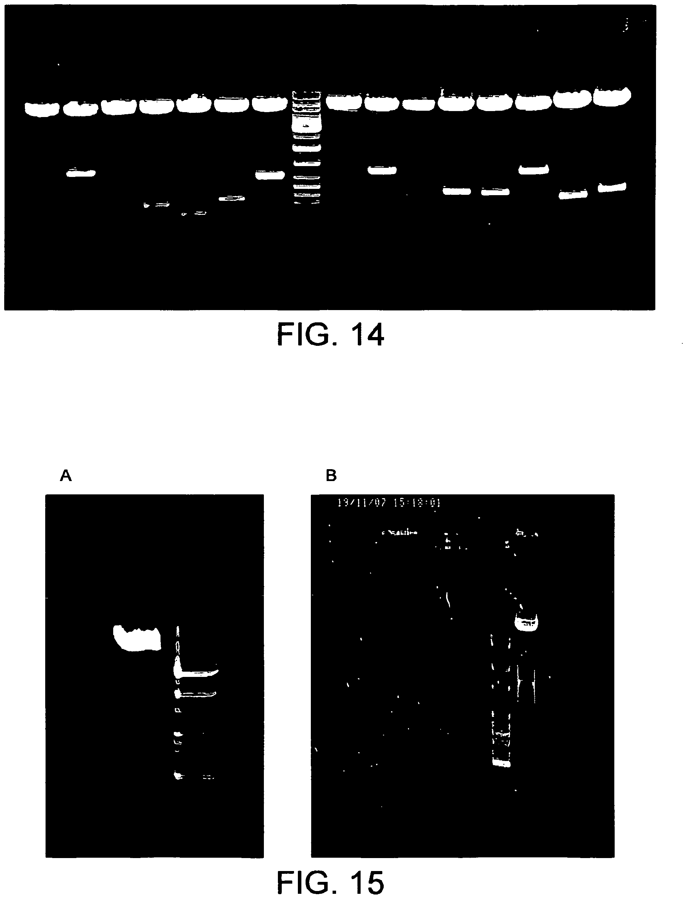

Representative brain UTR library according to the present invention is shown in figure 14.
Size selected cDNA and Cloned library Sfi I digested are shown in figures 15A and 15B respectively. Library was spiked with 20% MAFB-UTR plasmid.

### Selection Screen:

Transfected into MCF7 cells and MCF7 cells expressing mir130A.
Transfected cells were selected with zeocin
(~ 2000 colonies).
Genomic DNA was isolated and amount of plasmid MAFB-UTR was determined by qPCRPCR of UTRs present in MCF7 + library and MCF7 mir130A + library and Topo TA cloning for sequencing of individual clones.
MCF7 + library +20% MAFB transfected with mir10A, mir130A and shRNA against MAFB.
Selection in zeocin (reduction in MAFB).
Selection in Gancyclovir (MAFB enrichment and identification of mir10A and mir130A targets).

Although the present invention has been described in connection with specific preferred embodiments, it should be understood that the invention as claimed should not be unduly limited to such specific embodiments. Indeed, various modifications of the described modes for carrying out the invention which are apparent to those skilled in the art are intended to be within the scope of the following claims.

### Sequence Listing

SEQ ID NO: 1
   nucleic acid sequence of TK-PURO fusion 
ATGGCCTCGTACCCCGGCCATCAACACGCGTCTGCGTTCGACCAGGCTGCGCGTTCTCGCGGCCATAGC
AACCGACGTACGGCGTTGCGCCCTCGCCGGCAGCAAGAAGCCACGGAAGTCCGCCCGGAGCAGAAAATG
CCCACGCTACTGCGGGTTTATATAGACGGTCCCCACGGGATGGGGAAAACCACCACCACGCAACTGCTG
GTGGCCCTGGGTTCGCGCGACGATATCGTCTACGTACCCGAGCCGATGACTTACTGGCGGGTGCTGGGG
GCTTCCGAGACAATCGCGAACATCTACACCACACAACACCGCCTCGACCAGGGTGAGATATCGGCCGGG
GACGCGGCGGTGGTAATGACAAGCGCCCAGATAACAATGGGCATGCCTTATGCCGTGACCGACGCCGTT
CTGGCTCCTCATATCGGGGGGGAGGCTGGGAGCTCACATGCCCCGCCCCCGGCCCTCACCCTCATCTTC
GACCGCCATCCCATCGCCGCCCTCCTGTGCTACCCGGCCGCGCGGTACCTTATGGGCAGCATGACCCCC
CAGGCCGTGCTGGCGTTCGTGGCCCTCATCCCGCCGACCTTGCCCGGCACCAACATCGTGCTTGGGGCC
CTTCCGGAGGACAGACACATCGACCGCCTGGCCAAACGCCAGCGCCCCGGCGAGCGGCTGGACCTGGCT
ATGCTGGCTGCGATTCGCCGCGTTTACGGCTACTTGCCAATACGGTGCGGTATCTGCAGTGCGGCGGG
TCGTGGCGGGAGGACTGGGGACAGCTTTCGGGGACGGCCGTGCCGCCCCAGGGTGCCGAGCCCCAGAGC
AACGCGGGCCCACGACCCCATATCGGGGACACGTTATTTACCCTGTTTCGGGCCCCCGAGTTGCTGGCC
CCCAACGGCGACCTGTATAACGTGTTTGCCTGGGCCTTGACGTCTTGGCCCAAACGCCTCCGTTCCATG
CACGTCTTTATCCTGGATTACGACCAATCGCCCGCCGGCTGCCGGGACGCCCTGCTGCAACTTACCTCC
GGGATGGTCCAGACCCACGTCACCACCCCCGGCTCCATACCGACGATATGCGACCTGGCGCGCACGTTT
GCCCGAGAAATGAAGCTTACCATGACCGAGTACAAGCCCACGGTGCGCCTCGCCACCCGCGACGACGTC
CCCAGGGCCGTACGCACCCTCGCCGCCGCGTTCGCCGACTACCCCGCCACGCGCCACACCGTCGATCCG
GACCGCCACATCGAGCGGGTCACCGAGCTGCAAGAACTCTTCCTCACGCGCGTCGGGCTCGACATCGGC
AAGGTGTGGGTCGCGGACGACGGCGCCGCGGTGGCGGTCTGGACCACGCCGGAGAGCGTCGAAGCGGGG
GCGGTGTTCGCCGAGATCGGCCCGCGCATGGCCGAGTTGAGCGGTTCCCGGCTGGCCGCGCAGCAACAG
ATGGAAGGCCTCCTGGCGCCGCACCGGCCCAAGGAGCCCGCGTGGTTCCTGGCCACCGTCGGCGTCTCG
CCCGACCACCAGGGCAAGGGTCTGGGCAGCGCCGTCGTGCTCCCCGGAGTGGAGGCGGCCGAGCGCGCC
GGGGTGCCCGCCTTCCTGGAGACCTCCGCGCCCCGCAACCTCCCCTTCTACGAGCGGCTCGGCTTCACC
GTCACCGCCGACGTCGAGGTGCCCGAAGGACCGCGCACCTGGTGCATGACCCGCAAGCCCGGTGCCTGA
SEQ ID NO: 2
   nucleic acid sequence of plasmid backbone 
GCTAGCATCGATAAGAATTCCGGATCCTTAGGCCATTAAGGCCGGCCGCCTCGGCCCACTTCG
TGGGGTACCGAGCTCGAATTCACTGGCCGTCGTTTTACAACGTCGTGACTGGGAAAACCCTGGCGTTAC
CCAACTTAATCGCCTTGCAGCACATCCCCCTTTCGCCAGCTGGCGTAATAGCGAAGAGGCCCGCACCGA
TCGCCCTTCCCAACAGTTGCGTGGCCGAGGAGCAGGACTGACACGTGCTACGAGATTTCGATTCCACCG
CCGCCTTCTATGAAAGGTTGGGCTTCGGAATCGTTTTCCGGGACGCCGGCTGGATGATCCTCCAGCGCG
GGGATCTCATGCTGGAGTTCTTCGCCCACCCCAACTTGTTTATTGCAGCTTATAATGGTTACAAATAAA
GCAATAGCATCACAAATTTCACAAATAAAGCATTTTTTTCACTGCATTCTAGTTGTGGTTTGTCCAAAC
TCATCAATGTATCTTATCATGTCTGTATACCGTCGACCTCTAGCTAGAGCTTGGCGTAATCATGGTCAT
AGCTGTTTCCTGTGTGAAATTGTTATCCGCTCACAATTCCACACAACATACGAGCCGGAAGCATAAAGT
GTAAAGCCTGGGGTGCCTAATGAGTGAGCTAACTCACATTAATTGCGTTGCGCTCACTGCCCGCTTTCC
AGTCGGGAAACCTGTCGTGCCAGCTGCATTAATGAATCGGCCAACGCGCGGGGAGAGGCGGTTTGCGTA
TTGGGCGCTCTTCCGCTTCCTCGCTCACTGACTCGCTGCGCTCGGTCGTTCGGCTGCGGCGAGCGGTAT
CAGCTCACTCAAAGGCGGTAATACGGTTATCCACAGAATCAGGGGATAACGCAGGAAAGAACATGTGAG
CAAAAGGCCAGCAAAAGGCCAGGAACCGTAAAAAGGCCGCGTTGCTGGCGTTTTTCCATAGGCTCCGCC
CCCCTGACGAGCATCACAAAAATCGACGCTCAAGTCAGAGGTGGCGAAACCCGACAGGACTATAAAGAT
 
ACCAGGCGTTTCCCCCTGGAAGCTCCCTCGTGCGCTCTCCTGTTCCGACCCTGCCGCTTACCGGATACC
TGTCCGCCTTTCTCCCTTCGGGAAGCGTGGCGCTTTCTCAATGCTCACGCTGTAGGTATCTCAGTTCGG
TGTAGGTCGTTCGCTCCAAGCTGGGCTGTGTGCACGAACCCCCCGTTCAGCCCGACCGCTGCGCCTTAT
CCGGTAACTATCGTCTTGAGTCCAACCCGGTAAGACACGACTTATCGCCACTGGCAGCAGCCACTGGTA
ACAGGATTAGCAGAGCGAGGTATGTAGGCGGTGCTACAGAGTTCTTGAAGTGGTGGCCTAACTACGGCT
ACACTAGAAGGACAGTATTTGGTATCTGCGCTCTGCTGAAGCCAGTTACCTTCGGAAAAAGAGTTGGTA
GCTCTTGATCCGGCAAACAAACCACCGCTGGTAGCGGTGGTTTTTTTGTTTGCAAGCAGCAGATTACGC
GCAGAAAAAAAGGATCTCAAGAAGATCCTTTGATCTTTTCTACGGGGTCTGACGCTCAGTGGAACGAAA
ACTCACGTTAAGGGATTTTGGTCATGAGATTATCAAAAAGGATCTTCACCTAGATCCTTTTAAATTAAA
AATGAAGTTTTAAATCAATCTAAAGTATATATGAGTAAACTTGGTCTGACAGTTACCAATGCTTAATCA
GTGAGGCACCTATCTCAGCGATCTGTCTATTTCGTTCATCCATAGTTGCCTGACTCCCCGTCGTGTAGA
TAACTACGATACGGGAGGGCTTACCATCTGGCCCCAGTGCTGCAATGATACCGCGAGACCCACGCTCAC
CGGCTCCAGATTTATCAGCAATAAACCAGCCAGCCGGAAGGGCCGAGCGCAGAAGTGGTCCTGCAACTT
TATCCGCCTCCATCCAGTCTATTAATTGTTGCCGGGAAGCTAGAGTAAGTAGTTCGCCAGTTAATAGTT
TGCGCAACGTTGTTGCCATTGCTACAGGCATCGTGGTGTCACGCTCGTCGTTTGGTATGGCTTCATTCA
GCTCCGGTTCCCAACGATCAAGGCGAGTTACATGATCCCCCATGTTGTGCAAAAAAGCGGTTAGCTCCT
TCGGTCCTCCGATCGTTGTCAGAAGTAAGTTGGCCGCAGTGTTATCACTCATGGTTATGGCAGCACTGC
ATAATTCTCTTACTGTCATGCCATCCGTAAGATGCTTTTCTGTGACTGGTGAGTACTCAACCAAGTCAT
TCTGAGAATAGTGTATGCGGCGACCGAGTTGCTCTTGCCCGGCGTCAATACGGGATAATACCGCGCCAC
ATAGCAGAACTTTAAAAGTGCTCATCATTGGAAAACGTTCTTCGGGGCGAAAACTCTCAAGGATCTTAC
CGCTGTTGAGATCCAGTTCGATGTAACCCACTCGTGCACCCAACTGATCTTCAGCATCTTTTACTTTCA
CCAGCGTTTCTGGGTGAGCAAAAACAGGAAGGCAAAATGCCGCAAAAAAGGGAATAAGGGCGACACGGA
AATGTTGAATACTCATACTCTTCCTTTTTCAATATTATTGAAGCATTTATCAGGGTTATTGTCTCATGA
GCGGATACATATTTGAATGTATTTAGAAAAATAAACAAATAGGGGTTCCGCGCACATTTCCCCGAAAAG
TGCCACCTGACGTCGACGGATCGGGAGATCTCCCGATCCCCTATGGTCGACTCTCAGTACAATCTGCTC
TGATGCCGCATAGTTAAGCCAGTATCTGCTCCCTGCTTGTGTGTTGGAGGTCGCTGAGTAGTGCGCGAG
CAAAATTTAAGCTACAACAAGGCAAGGCTTGACCGACAATTGCATGAAGAATCTGCTTAGGGTTAGGCG
TTTTGCGCTGCTTCGCGATGTACGGGCCAGATATACGCGTTGACATTGATTATTGACTAGTTATTAATA
GTAATCAATTACGGGGTCATTAGTTCATAGCCCATATATGGAGTTCCGCGTTACATAACTTACGGTAAA
TGGCCCGCCTGGCTGACCGCCCAACGACCCCCGCCCATTGACGTCAATAATGACGTATGTTCCCATAGT
AACGCCAATAGGGACTTTCCATTGACGTCAATGGGTGGACTATTTACGGTAAACTGCCCACTTGGCAGT
ACATCAAGTGTATCATATGCCAAGTACGCCCCCTATTGACGTCAATGACGGTAAATGGCCCGCCTGGCA
TTATGCCCAGTACATGACCTTATGGGACTTTCCTACTTGGCAGTACATCTACGTATTAGTCATCGCTAT
TACCATGGTGATGCGGTTTTGGCAGTACATCAATGGGCGTGGATAGCGGTTTGACTCACGGGGATTTCC
AAGTCTCCACCCCATTGACGTCAATGGGAGTTTGTTTTGGCACCAAAATCAACGGGACTTTCCAAAATG
TCGTAACAACTCCGCCCCATTGACGCAAATGGGCGGTAGGCGTGTACGGTGGGAGGTCTATATAAGCAG
AGCTCTCTGGCTAACTAGAGAACCCACTGCTTACTGGCTTATCGAAATTAATACGACTCACTATAGGGA
GACCCAAGCTG
SEQ ID NO: 3
   nucleic acid sequence of Hygro/TK fusion 
ATGGGTAAAAAGCCTGAACTCACCGCGACGTCTGTCGAGAAGTTTCTGATCGAAAAGT
TCGACAGCGTCTCCGACCTGATGCAGCTCTCGGAGGGCGAAGAATCTCGTGCTTTCAG
CTTCGATGTAGGAGGGCGTGGATATGTCCTGCGGGTAAATAGCTGCGCCGATGGTTTC
TACAAAGATCGTTATGTTTATCGGCACTTTGCATCGGCCGCGCTCCCGATTCCGGAAG
TGCTTGACATTGGGGAATTCAGCGAGAGCCTGACCTATTGCATCTCCCGCCGTGCACA
GGGTGTCACGTTGCAAGACCTGCCTGAAACCGAACTGCCCGCTGTTCTGCAGCCGGTC
GCGGAGGCCATGGATGCGATCGCTGCGGCCGATCTTAGCCAGACGAGCGGGTTCGGCC
CATTCGGACCGCAAGGAATCGGTCAATACACTACATGGCGTGATTTCATATGCGCGAT
TGCTGATCCCCATGTGTATCACTGGCAAACTGTGATGGACGACACCGTCAGTGCGTCC
GTCGCGCAGGCTCTCGATGAGCTGATGCTTTGGGCCGAGGACTGCCCCGAAGTCCGGC
ACCTCGTGCACGCGGATTTCGGCTCCAACAATGTCCTGACGGACAATGGCCGCATAAC
AGCGGTCATTGACTGGAGCGAGGCGATGTTCGGGGATTCCCAATACGAGGTCGCCAAC
ATCTTCTTCTGGAGGCCGTGGTTGGCTTGTATGGAGCAGCAGACGCGCTACTTCGAGC
GGAGGCATCCGGAGCTTGCAGGATCGCCGCGGCTCCGGGCGTATATGCTCCGCATTGG
TCTTGACCAACTCTATCAGAGCTTGGTTGACGGCAATTTCGATGATGCAGCTTGGGCG
CAGGGTCGATGCGACGCAATCGTCCGATCCGGAGCCGGGACTGTCGGGCGTACACAAA
TCGCCCGCAGAAGCGCGGCCGTCTGGACCGATGGCTGTGTAGAAGTCGCGTCTGCGTT
CGACCAGGCTGCGCGTTCTCGCGGCCATAGCAACCGACGTACGGCGTTGCGCCCTCGC
CGGCAGCAAGAAGCCACGGAAGTCCGCCCGGAGCAGAAAATGCCCACGCTACTGCGGG
TTTATATAGACGGTCCCCACGGGATGGGGAAAACCACCACCACGCAACTGCTGGTGGC
CCTGGGTTCGCGCGACGATATCGTCTACGTACCCGAGCCGATGACTTACTGGCGGGTG
CTGGGGGCTTCCGAGACAATCGCGAACATCTACACCACACAACACCGCCTCGACCAGG
GTGAGATATCGGCCGGGGACGCGGCGGTGGTAATGACAAGCGCCCAGATAACAATGGG
CATGCCTTATGCCGTGACCGACGCCGTTCTGGCTCCTCATATCGGGGGGGAGGCTGGG
AGCTCACATGCCCCGCCCCCGGCCCTCACCCTCATCTTCGACCGCCATCCCATCGCCG
CCCTCCTGTGCTACCCGGCCGCGCGGTACCTTATGGGCAGCATGACCCCCCAGGCCGT
GCTGGCGTTCGTGGCCCTCATCCCGCCGACCTTGCCCGGCACCAACATCGTGCTTGGG
GCCCTTCCGGAGGACAGACACATCGACCGCCTGGCCAAACGCCAGCGCCCCGGCGAGC
GGCTGGACCTGGCTATGCTGGCTGCGATTCGCCGCGTTTACGGGCTACTTGCCAATAC
GGTGCGGTATCTGCAGTGCGGCGGGTCGTGGCGGGAGGACTGGGGACAGCTTTCGGGG
ACGGCCGTGCCGCCCCAGGGTGCCGAGCCCCAGAGCAACGCGGGCCCACGACCCCATA
TCGGGGACACGTTATTTACCCTGTTTCGGGCCCCCGAGTTGCTGGCCCCCAACGGCGA
CCTGTATAACGTGTTTGCCTGGGCCTTGGACGTCTTGGCCAAACGCCTCCGTTCCATG
CACGTCTTTATCCTGGATTACGACCAATCGCCCGCCGGCTGCCGGGACGCCCTGCTGC
AACTTACCTCCGGGATGGTCCAGACCCACGTCACCACCCCCGGCTCCATACCGACGAT
ATGCGACCTGGCGCGCACGTTTGCCCGAGAAATGAAGCTTCGATAA
SEQ ID NO: 4
   nucleic acid sequence of TKzeo fusion 
ATGGCTTCGTACCCCGGCCATCAACACGCGTCTGCGTTCGACCAGGCTGCGCG
TTCTCGCGGCCATAGCAACCGACGTACGGCGTTGCGCCCTCGCCGGCAGCAAGAAGCC
ACGGAAGTCCGCCCGGAGCAGAAAATGCCCACGCTACTGCGGGTTTATATAGACGGTC
CCCACGGGATGGGGAAAACCACCACCACGCAACTGCTGGTGGCCCTGGGTTCGCGCGA
 
CGATATCGTCTACGTACCCGAGCCGATGACTTACTGGCGGTGCTGGGGCTTCCGAG
ACAATCGCGAACATCTACACCACACAACACCGCCTCGACCAGGGTGAGATATCGGCCG
GGGACGCGGCGGTGGTAATGACAAGCGCCCAGATAACAATGGGCATGCCTTATGCCGT
GACCGACGCCGTTCTGGCTCCTCATATCGGGGGGAGGCTGGAGCTCACATGCCCCG
CCCCCGGCCCTCACCCTCATCTTCGACCGCCATCCCATCGCCGCCCTCCTGTGCTACC
CGGCCGCGCGGTACCTTATGGGCAGCATGACCCCCCAGGCCGTGCTGGCGTTCGTGGC
CCTCATCCCGCCGACCTTGCCCGGCACCAACATCGTGCTTGGGGCCCTTCCGGAGGAC
AGACACATCGACCGCCTGGCCAAACGCCAGCGCCCCGGCGAGCGGCTGGACCTGGCTA
TGCTGGCTGCGATTCGCCGCGTTTACGGGCTACTTGCCAATACGGTGCGGTATCTGCA
GTGCGGCGGGTCGTGGCGGGAGGACTGGGGACAGCTTTCGGGGACGGCCGTGCCGCCC
CAGGGTGCCGAGCCCCAGAGCAACGCGGGCCCACGACCCCATATCGGGGACACGTTAT
TTACCCTGTTTCGGGCCCCCGAGTTGCTGGCCCCCAACGGCGACCTGTATAACGTGTT
TGCCTGGGCCTTGGACGTCTTGGCCAAACGCCTCCGTTCCATGCACGTCTTTATCCTG
GATTACGACCAATCGCCCGCCGGCTGCCGGGACGCCCTGCTGCAACTTACCTCCGGGA
TGGTCCAGACCCACGTCACCACCCCCGGCTCCATACCGACGATATGCGACCTGGCGCG
CACGTTTGCCCGAGAGATGATCAGCGGAGCTAATGGCGTCATGGCCAAGTTGACCAGT
GCCGTTCCGGTGCTCACCGCGCGCGACGTCGCCGGAGCGGTCGAGTTCTGGACCGACC
GGCTCGGGTTCTCCCGGGACTTCGTGGAGGACGACTTCGCCGGTGTGGTCCGGGACGA
CGTGACCCTGTTCATCAGCGCGGTCCAGGACCAGGTGGTGCCGGACAACACCCTGGCC
TGGGTGTGGGTGCGCGGCCTGGACGAGCTGTACGCCGAGTGGTCGGAGGTCGTGTCCA
CGAACTTCCGGGACGCCTCCGGGCCGGCCATGACCGAGATCGGCGAGCAGCCGTGGGG
GCGGGAGTTCGCCCTGCGCGACCCGGCCGGCAACTGCGTGCACTTCGTGGCCGAGGAG
CAGGACTGA
SEQ ID NO: 5
   p3'HYTK 
CCTAGGCTTTGCAAAAGCTTGGCCACATGGGTAAAAAGCCTGAA
CTCACCGCGACGTCTGTCGAGAAGTTTCTGATCGAAAAGTTCGACAGCGTC
TCCGACCTGATGCAGCTCTCGGAGGGCGAAGAATCTCGTGCTTTCAGCTTC
GATGTAGGAGGGCGTGGATATGTCCTGCGGGTAAATAGCTGCGCCGATGG
TTTCTACAAAGATCGTTATGTTTATCGGCACTTTGCATCGGCCGCGCTCCCG
ATTCCGGAAGTGCTTGACATTGGGGAATTCAGCGAGAGCCTGACCTATTGC
ATCTCCCGCCGTGCACAGGGTGTCACGTTGCAAGACCTGCCTGAAACCGAA
 
CTGCCCGCTGTTCTGCAGCCGGTCGCGGAGGCCATGGATGCGATCGCTGCG
GCCGATCTTAGCCAGACGAGCGGGTTCGGCCCATTCGGACCGCAAGGAAT
CGGTCAATACACTACATGGCGTGATTTCATATGCGCGATTGCTGATCCCCA
TGTGTATCACTGGCAAACTGTGATGGACGACACCGTCAGTGCGTCCGTCGC
GCAGGCTCTCGATGAGCTGATGCTTTGGGCCGAGGACTGCCCCGAAGTCCG
GCACCTCGTGCACGCGGATTTCGGCTCCAACAATGTCCTGACGGACAATGG
CCGCATAACAGCGGTCATTGACTGGAGCGAGGCGATGTTCGGGGATTCCC
AATACGAGGTCGCCAACATCTTCTTCTGGAGGCCGTGGTTGGCTTGTATGG
AGCAGCAGACGCGCTACTTCGAGCGGAGGCATCCGGAGCTTGCAGGATCG
CCGCGGCTCCGGGCGTATATGCTCCGCATTGGTCTTGACCAACTCTATCAG
AGCTTGGTTGACGGCAATTTCGATGATGCAGCTTGGGCGCAGGGTCGATGC
GACGCAATCGTCCGATCCGGAGCCGGGACTGTCGGGCGTACACAAATCGC
CCGCAGAAGCGCGGCCGTCTGGACCGATGGCTGTGTAGAAGTCGCGTCTG
CGTTCGACCAGGCTGCGCGTTCTCGCGGCCATAGCAACCGACGTACGGCGT
TGCGCCCTCGCCGGCAGCAAGAAGCCACGGAAGTCCGCCCGGAGCAGAAA
ATGCCCACGCTACTGCGGGTTTATATAGACGGTCCCCACGGATGGGGAA
AACCACCACCACGCAACTGCTGGTGGCCCTGGGTTCGCGCGACGATATCGT
CTACGTACCCGAGCCGATGACTTACTGGCGGGTGCTGGGGGCTTCCGAGAC
AATCGCGAACATCTACACCACACAACACCGCCTCGACCAGGGTGAGATAT
CGGCCGGGGACGCGGCGGTGGTAATGACAAGCGCCCAGATAACAATGGGC
ATGCCTTATGCCGTGACCGACGCCGTTCTGGCTCCTCATATCGGGGGGGAG
GCTGGGAGCTCACATGCCCCGCCCCCGGCCCTCACCCTCATCTTCGACCGC
CATCCCATCGCCGCCCTCCTGTGCTACCCGGCCGCGCGGTACCTTATGGGC
AGCATGACCCCCCAGGCCGTGCTGGCGTTCGTGGCCCTCATCCCGCCGACC
TTGCCCGGCACCAACATCGTGCTTGGGGCCCTTCCGGAGGACAGACACATC
GACCGCCTGGCCAAACGCCAGCGCCCCGGCGAGCGGCTGGACCTGGCTAT
GCTGGCTGCGATTCGCCGCGTTTACGGGCTACTTGCCAATACGGTGCGGTA
TCTGCAGTGCGGCGGGTCGTGGCGGGAGGACTGGGGACAGCTTTCGGGGA
CGGCCGTGCCGCCCCAGGGTGCCGAGCCCCAGAGCAACGCGGGCCCACGA
CCCCATATCGGGGACACGTTATTTACCCTGTTTCGGGCCCCCGAGTTGCTG
GCCCCCAACGGCGACCTGTATAACGTGTTTGCCTGGGCCTTGACGTCTTGG
CCCAAACGCCTCCGTTCCATGCACGTCTTTATCCTGGATTACGACCAATCG
 
CCCGCCGGCTGCCGGACGCCCTGCTGCAACTTACCTCCGGGATGGTCCAG
ACCCACGTCACCACCCCCGGCTCCATACCGACGATATGCGACCTGGCGCGC
ACGTTTGCCCGAGAAATGAAGCTTCGATAAGAATTCCGGATCCTTAGGCCA
TTAAGGCCGGCCGCCTCGGCCCACTTCGTGGGGTACCGAGCTCGAATTCAC
TGGCCGTCGTTTTACAACGTCGTGACTGGGAAAACCCTGGCGTTACCCAAC
TTAATCGCCTTGCAGCACATCCCCCTTTCGCCAGCTGGCGTAATAGCGAAG
AGGCCCGCACCGATCGCCCTTCCCAACAGTTGCGTGGCCGAGGAGCAGGA
CTGACACGTGCTACGAGATTTCGATTCCACCGCCGCCTTCTATGAAAGGTT
GGGCTTCGGAATCGTTTTCCGGGACGCCGGCTGGATGATCCTCCAGCGCGG
GGATCTCATGCTGGAGTTCTTCGCCCACCCCAACTTGTTTATTGCAGCTTAT
AATGGTTACAAATAAAGCAATAGCATCACAAATTTCACAAATAAAGCATT
TTTTTCACTGCATTCTAGTTGTGGTTTGTCCAAACTCATCAATGTATCTTAT
CATGTCTGTATACCGTCGACCTCTAGCTAGAGCTTGGCGTAATCATGGTCA
TAGCTGTTTCCTGTGTGAAATTGTTATCCGCTCACAATTCCACACAACATAC
GAGCCGGAAGCATAAAGTGTAAAGCCTGGGGTGCCTAATGAGTGAGCTAA
CTCACATTAATTGCGTTGCGCTCACTGCCCGCTTTCCAGTCGGGAAACCTG
TCGTGCCAGCTGCATTAATGAATCGGCCAACGCGCGGGGAGAGGCGGTTT
GCGTATTGGGCGCTCTTCCGCTTCCTCGCTCACTGACTCGCTGCGCTCGGTC
GTTCGGCTGCGGCGAGCGGTATCAGCTCACTCAAAGGCGGTAATACGGTT
ATCCACAGAATCAGGGGATAACGCAGGAAAGAACATGTGAGCAAAAGGC
CAGCAAAAGGCCAGGAACCGTAAAAAGGCCGCGTTGCTGGCGTTTTTCCA
TAGGCTCCGCCCCCCTGACGAGCATCACAAAAATCGACGCTCAAGTCAGA
GGTGGCGAAACCCGACAGGACTATAAAGATACCAGGCGTTTCCCCCTGGA
AGCTCCCTCGTGCGCTCTCCTGTTCCGACCCTGCCGCTTACCGGATACCTGT
CCGCCTTTCTCCCTTCGGGAAGCGTGGCGCTTTCTCAATGCTCACGCTGTAG
GTATCTCAGTTCGGTGTAGGTCGTTCGCTCCAAGCTGGGCTGTGTGCACGA
ACCCCCCGTTCAGCCCGACCGCTGCGCCTTATCCGGTAACTATCGTCTTGA
GTCCAACCCGGTAAGACACGACTTATCGCCACTGGCAGCAGCCACTGGTA
ACAGGATTAGCAGAGCGAGGTATGTAGGCGGTGCTACAGAGTTCTTGAAG
TGGTGGCCTAACTACGGCTACACTAGAAGGACAGTATTTGGTATCTGCGCT
CTGCTGAAGCCAGTTACCTTCGGAAAAAGAGTTGGTAGCTCTTGATCCGGC
AAACAAACCACCGCTGGTAGCGGTGGTTTTTTTGTTTGCAAGCAGCAGATT
 
ACGCGCAGAAAAAAAGGATCTCAAGAAGATCCTTTGATCTTTTCTACGGG
GTCTGACGCTCAGTGGAACGAAAACTCACGTTAAGGGATTTTGGTCATGAG
ATTATCAAAAAGGATCTTCACCTAGATCCTTTTAAATTAAAAATGAAGTTT
TAAATCAATCTAAAGTATATATGAGTAAACTTGGTCTGACAGTTACCAATG
CTTAATCAGTGAGGCACCTATCTCAGCGATCTGTCTATTTCGTTCATCCATA
GTTGCCTGACTCCCCGTCGTGTAGATAACTACGATACGGGAGGGCTTACCA
TCTGGCCCCAGTGCTGCAATGATACCGCGAGACCCACGCTCACCGGCTCCA
GATTTATCAGCAATAAACCAGCCAGCCGGAAGGGCCGAGCGCAGAAGTGG
TCCTGCAACTTTATCCGCCTCCATCCAGTCTATTAATTGTTGCCGGGAAGCT
AGAGTAAGTAGTTCGCCAGTTAATAGTTTGCGCAACGTTGTTGCCATTGCT
ACAGGCATCGTGGTGTCACGCTCGTCGTTTGGTATGGCTTCATTCAGCTCC
GGTTCCCAACGATCAAGGCGAGTTACATGATCCCCCATGTTGTGCAAAAAA
GCGGTTAGCTCCTTCGGTCCTCCGATCGTTGTCAGAAGTAAGTTGGCCGCA
GTGTTATCACTCATGGTTATGGCAGCACTGCATAATTCTCTTACTGTCATGC
CATCCGTAAGATGCTTTTCTGTGACTGGTGAGTACTCAACCAAGTCATTCT
GAGAATAGTGTATGCGGCGACCGAGTTGCTCTTGCCCGGCGTCAATACGG
GATAATACCGCGCCACATAGCAGAACTTTAAAAGTGCTCATCATTGGAAA
ACGTTCTTCGGGGCGAAAACTCTCAAGGATCTTACCGCTGTTGAGATCCAG
TTCGATGTAACCCACTCGTGCACCCAACTGATCTTCAGCATCTTTTACTTTC
ACCAGCGTTTCTGGGTGAGCAAAAACAGGAAGGCAAAATGCCGCAAAAAA
GGGAATAAGGGCGACACGGAAATGTTGAATACTCATACTCTTCCTTTTTCA
ATATTATTGAAGCATTTATCAGGGTTATTGTCTCATGAGCGGATACATATTT
GAATGTATTTAGAAAAATAAACAAATAGGGGTTCCGCGCACATTTCCCCG
AAAAGTGCCACCTGACGTCGACGGATCGGGAGATCTCCCGATCCCCTATG
GTCGACTCTCAGTACAATCTGCTCTGATGCCGCATAGTTAAGCCAGTATCT
GCTCCCTGCTTGTGTGTTGGAGGTCGCTGAGTAGTGCGCGAGCAAAATTTA
AGCTACAACAAGGCAAGGCTTGACCGACAATTGCATGAAGAATCTGCTTA
GGGTTAGGCGTTTTGCGCTGCTTCGCGATGTACGGGCCAGATATACGCGTT
GACATTGATTATTGACTAGTTATTAATAGTAATCAATTACGGGGTCATTAG
TTCATAGCCCATATATGGAGTTCCGCGTTACATAACTTACGGTAAATGGCC
CGCCTGGCTGACCGCCCAACGACCCCCGCCCATTGACGTCAATAATGACGT
ATGTTCCCATAGTAACGCCAATAGGGACTTTCCATTGACGTCAATGGGTGG
 
ACTATTTACGGTAAACTGCCCACTTGGCAGTACATCAAGTGTATCATATGC
CAAGTACGCCCCCTATTGACGTCAATGACGGTAAATGGCCCGCCTGGCATT
ATGCCCAGTACATGACCTTATGGGACTTTCCTACTTGGCAGTACATCTACG
TATTAGTCATCGCTATTACCATGGTGATGCGGTTTTGGCAGTACATCAATG
GGCGTGGATAGCGGTTTGACTCACGGGGATTTCCAAGTCTCCACCCCATTG
ACGTCAATGGGAGTTTGTTTTGGCACCAAAATCAACGGGACTTTCCAAAAT
GTCGTAACAACTCCGCCCCATTGACGCAAATGGGCGGTAGGCGTGTACGG
TGGGAGGTCTATATAAGCAGAGCTCTCTGGCTAACTAGAGAACCCACTGCT
TACTGGCTTATCGAAATTAATACGACTCACTATAGGGAGACCCAAGCTGGC
TAG
SEQ ID NO: 6
   p3'TKPUR 
GCTAGCTTATCGCATGGCCTCGTACCCCGGCCATCAACACGCGTCTG
CGTTCGACCAGGCTGCGCGTTCTCGCGGCCATAGCAACCGACGTACGGCGT
TGCGCCCTCGCCGGCAGCAAGAAGCCACGGAAGTCCGCCCGGAGCAGAAA
ATGCCCACGCTACTGCGGGTTTATATAGACGGTCCCCACGGATGGGGAA
AACCACCACCACGCAACTGCTGGTGGCCCTGGGTTCGCGCGACGATATCGT
CTACGTACCCGAGCCGATGACTTACTGGCGGGTGCTGGGGGCTTCCGAGAC
AATCGCGAACATCTACACCACACAACACCGCCTCGACCAGGGTGAGATAT
CGGCCGGGGACGCGGCGGTGGTAATGACAAGCGCCCAGATAACAATGGGC
ATGCCTTATGCCGTGACCGACGCCGTTCTGGCTCCTCATATCGGGGGGGAG
GCTGGGAGCTCACATGCCCCGCCCCCGGCCCTCACCCTCATCTTCGACCGC
CATCCCATCGCCGCCCTCCTGTGCTACCCGGCCGCGCGGTACCTTATGGGC
AGCATGACCCCCCAGGCCGTGCTGGCGTTCGTGGCCCTCATCCCGCCGACC
TTGCCCGGCACCAACATCGTGCTTGGGGCCCTTCCGGAGGACAGACACATC
GACCGCCTGGCCAAACGCCAGCGCCCCGGCGAGCGGCTGGACCTGGCTAT
GCTGGCTGCGATTCGCCGCGTTTACGGGCTACTTGCCAATACGGTGCGGTA
TCTGCAGTGCGGCGGGTCGTGGCGGGAGGACTGGGGACAGCTTTCGGGGA
CGGCCGTGCCGCCCCAGGGTGCCGAGCCCCAGAGCAACGCGGGCCCACGA
CCCCATATCGGGGACACGTTATTTACCCTGTTTCGGCCCCCGAGTTGCTG
GCCCCCAACGGCGACCTGTATAACGTGTTTGCCTGGGCCTTGACGTCTTGG
CCCAAACGCCTCCGTTCCATGCACGTCTTTATCCTGGATTACGACCAATCG
 
CCCGCCGGCTGCCGGGACGCCCTGCTGCAACTTACCTCCGGGATGGTCCAG
ACCCACGTCACCACCCCCGGCTCCATACCGACGATATGCGACCTGGCGCGC
ACGTTTGCCCGAGAAATGAAGCTTACCATGACCGAGTACAAGCCCACGGT
GCGCCTCGCCACCCGCGACGACGTCCCCAGGGCCGTACGCACCCTCGCCGC
CGCGTTCGCCGACTACCCCGCCACGCGCCACACCGTCGATCCGGACCGCCA
CATCGAGCGGGTCACCGAGCTGCAAGAACTCTTCCTCACGCGCGTCGGGCT
CGACATCGGCAAGGTGTGGGTCGCGGACGACGGCGCCGCGGTGGCGGTCT
GGACCACGCCGGAGAGCGTCGAAGCGGGGGCGGTGTTCGCCGAGATCGGC
CCGCGCATGGCCGAGTTGAGCGGTTCCCGGCTGGCCGCGCAGCAACAGAT
GGAAGGCCTCCTGGCGCCGCACCGGCCCAAGGAGCCCGCGTGGTTCCTGG
CCACCGTCGGCGTCTCGCCCGACCACCAGGGCAAGGGTCTGGGCAGCGCC
GTCGTGCTCCCCGGAGTGGAGGCGGCCGAGCGCGCCGGGGTGCCCGCCTT
CCTGGAGACCTCCGCGCCCCGCAACCTCCCCTTCTACGAGCGGCTCGGCTT
CACCGTCACCGCCGACGTCGAGGTGCCCGAAGGACCGCGCACCTGGTGCA
TGACCCGCAAGCCCGGTGCCTGACGCCCGCCCCACGACCCGCAGCGCCCG
ACCGAAAGGAGCGCACGACCCCATGCATCGATAAGAATTCCGGATCCTTA
GGCCATTAAGGCCGGCCGCCTCGGCCCACTTCGTGGGGTACCGAGCTCGA
ATTCACTGGCCGTCGTTTTACAACGTCGTGACTGGGAAAACCCTGGCGTTA
CCCAACTTAATCGCCTTGCAGCACATCCCCCTTTCGCCAGCTGGCGTAATA
GCGAAGAGGCCCGCACCGATCGCCCTTCCCAACAGTTGCGTGGCCGAGGA
GCAGGACTGACACGTGCTACGAGATTTCGATTCCACCGCCGCCTTCTATGA
AAGGTTGGGCTTCGGAATCGTTTTCCGGGACGCCGGCTGGATGATCCTCCA
GCGCGGGGATCTCATGCTGGAGTTCTTCGCCCACCCCAACTTGTTTATTGC
AGCTTATAATGGTTACAAATAAAGCAATAGCATCACAAATTTCACAAATA
AAGCATTTTTTTCACTGCATTCTAGTTGTGGTTTGTCCAAACTCATCAATGT
ATCTTATCATGTCTGTATACCGTCGACCTCTAGCTAGAGCTTGGCGTAATC
ATGGTCATAGCTGTTTCCTGTGTGAAATTGTTATCCGCTCACAATTCCACAC
AACATACGAGCCGGAAGCATAAAGTGTAAAGCCTGGGGTGCCTAATGAGT
GAGCTAACTCACATTAATTGCGTTGCGCTCACTGCCCGCTTTCCAGTCGGG
AAACCTGTCGTGCCAGCTGCATTAATGAATCGGCCAACGCGCGGGGAGAG
GCGGTTTGCGTATTGGGCGCTCTTCCGCTTCCTCGCTCACTGACTCGCTGCG
CTCGGTCGTTCGGCTGCGGCGAGCGGTATCAGCTCACTCAAAGGCGGTAAT
 
ACGGTTATCCACAGAATCAGGGGATAACGCAGGAAAGAACATGTGAGCAA
AAGGCCAGCAAAAGGCCAGGAACCGTAAAAAGGCCGCGTTGCTGGCGTTT
TTCCATAGGCTCCGCCCCCCTGACGAGCATCACAAAAATCGACGCTCAAGT
CAGAGGTGGCGAAACCCGACAGGACTATAAAGATACCAGGCGTTTCCCCC
TGGAAGCTCCCTCGTGCGCTCTCCTGTTCCGACCCTGCCGCTTACCGGATA
CCTGTCCGCCTTTCTCCCTTCGGGAAGCGTGGCGCTTTCTCAATGCTCACGC
TGTAGGTATCTCAGTTCGGTGTAGGTCGTTCGCTCCAAGCTGGGCTGTGTG
CACGAACCCCCCGTTCAGCCCGACCGCTGCGCCTTATCCGGTAACTATCGT
CTTGAGTCCAACCCGGTAAGACACGACTTATCGCCACTGGCAGCAGCCACT
GGTAACAGGATTAGCAGAGCGAGGTATGTAGGCGGTGCTACAGAGTTCTT
GAAGTGGTGGCCTAACTACGGCTACACTAGAAGGACAGTATTTGGTATCTG
CGCTCTGCTGAAGCCAGTTACCTTCGGAAAAAGAGTTGGTAGCTCTTGATC
CGGCAAACAAACCACCGCTGGTAGCGGTGGTTTTTTTGTTTGCAAGCAGCA
GATTACGCGCAGAAAAAAAGGATCTCAAGAAGATCCTTTGATCTTTTCTAC
GGGGTCTGACGCTCAGTGGAACGAAAACTCACGTTAAGGGATTTTGGTCAT
GAGATTATCAAAAAGGATCTTCACCTAGATCCTTTTAAATTAAAAATGAAG
TTTTAAATCAATCTAAAGTATATATGAGTAAACTTGGTCTGACAGTTACCA
ATGCTTAATCAGTGAGGCACCTATCTCAGCGATCTGTCTATTTCGTTCATCC
ATAGTTGCCTGACTCCCCGTCGTGTAGATAACTACGATACGGGAGGGCTTA
CCATCTGGCCCCAGTGCTGCAATGATACCGCGAGACCCACGCTCACCGGCT
CCAGATTTATCAGCAATAAACCAGCCAGCCGGAAGGGCCGAGCGCAGAAG
TGGTCCTGCAACTTTATCCGCCTCCATCCAGTCTATTAATTGTTGCCGGGAA
GCTAGAGTAAGTAGTTCGCCAGTTAATAGTTTGCGCAACGTTGTTGCCATT
GCTACAGGCATCGTGGTGTCACGCTCGTCGTTTGGTATGGCTTCATTCAGC
TCCGGTTCCCAACGATCAAGGCGAGTTACATGATCCCCCATGTTGTGCAAA
AAAGCGGTTAGCTCCTTCGGTCCTCCGATCGTTGTCAGAAGTAAGTTGGCC
GCAGTGTTATCACTCATGGTTATGGCAGCACTGCATAATTCTCTTACTGTCA
TGCCATCCGTAAGATGCTTTTCTGTGACTGGTGAGTACTCAACCAAGTCAT
TCTGAGAATAGTGTATGCGGCGACCGAGTTGCTCTTGCCCGGCGTCAATAC
GGGATAATACCGCGCCACATAGCAGAACTTTAAAAGTGCTCATCATTGGA
AAACGTTCTTCGGGGCGAAAACTCTCAAGGATCTTACCGCTGTTGAGATCC
AGTTCGATGTAACCCACTCGTGCACCCAACTGATCTTCAGCATCTTTTACTT
 
TCACCAGCGTTTCTGGGTGAGCAAAAACAGGAAGGCAAAATGCCGCAAAA
AAGGGAATAAGGGCGACACGGAAATGTTGAATACTCATACTCTTCCTTTTT
CAATATTATTGAAGCATTTATCAGGGTTATTGTCTCATGAGCGGATACATA
TTTGAATGTATTTAGAAAAATAAACAAATAGGGGTTCCGCGCACATTTCCC
CGAAAAGTGCCACCTGACGTCGACGGATCGGGAGATCTCCCGATCCCCTAT
GGTCGACTCTCAGTACAATCTGCTCTGATGCCGCATAGTTAAGCCAGTATC
TGCTCCCTGCTTGTGTGTTGGAGGTCGCTGAGTAGTGCGCGAGCAAAATTT
AAGCTACAACAAGGCAAGGCTTGACCGACAATTGCATGAAGAATCTGCTT
AGGGTTAGGCGTTTTGCGCTGCTTCGCGATGTACGGGCCAGATATACGCGT
TGACATTGATTATTGACTAGTTATTAATAGTAATCAATTACGGGGTCATTA
GTTCATAGCCCATATATGGAGTTCCGCGTTACATAACTTACGGTAAATGGC
CCGCCTGGCTGACCGCCCAACGACCCCCGCCCATTGACGTCAATAATGACG
TATGTTCCCATAGTAACGCCAATAGGGACTTTCCATTGACGTCAATGGGTG
GACTATTTACGGTAAACTGCCCACTTGGCAGTACATCAAGTGTATCATATG
CCAAGTACGCCCCCTATTGACGTCAATGACGGTAAATGGCCCGCCTGGCAT
TATGCCCAGTACATGACCTTATGGGACTTTCCTACTTGGCAGTACATCTAC
GTATTAGTCATCGCTATTACCATGGTGATGCGGTTTTGGCAGTACATCAAT
GGGCGTGGATAGCGGTTTGACTCACGGGGATTTCCAAGTCTCCACCCCATT
GACGTCAATGGGAGTTTGTTTTGGCACCAAAATCAACGGGACTTTCCAAAA
TGTCGTAACAACTCCGCCCCATTGACGCAAATGGGCGGTAGGCGTGTACG
GTGGGAGGTCTATATAAGCAGAGCTCTCTGGCTAACTAGAGAACCCACTG
CTTACTGGCTTATCGAAATTAATACGACTCACTATAGGGAGACCCAAGCTG
SEQ ID NO: 7
   p3'TKZEO

CATGGCTTCGTACCCCGGCCATCAACACGCGTCTGCGTTCGACCAG
GCTGCGCGTTCTCGCGGCCATAGCAACCGACGTACGGCGTTGCGCCCTCGC
CGGCAGCAAGAAGCCACGGAAGTCCGCCCGGAGCAGAAAATGCCCACGCT
ACTGCGGGTTTATATAGACGGTCCCCACGGATGGGAAAACCACCACCA
CGCAACTGCTGGTGGCCCTGGGTTCGCGCGACGATATCGTCTACGTACCCG
AGCCGATGACTTACTGGCGGGTGCTGGGGGCTTCCGAGACAATCGCGAAC
ATCTACACCACACAACACCGCCTCGACCAGGGTGAGATATCGGCCGGGGA
CGCGGCGGTGGTAATGACAAGCGCCCAGATAACAATGGGCATGCCTTATG

CCGTGACCGACGCCGTTCTGGCTCCTCATATCGGGGGGAGGCTGGGAGCT
CACATGCCCCGCCCCCGGCCCTCACCCTCATCTTCGACCGCCATCCCATCG
CCGCCCTCCTGTGCTACCCGGCCGCGGTACCTTATGGGCAGCATGACCC
CCCAGGCCGTGCTGGCGTTCGTGGCCCTCATCCCGCCGACCTTGCCCGGCA
CCAACATCGTGCTTGGGGCCCTTCCGGAGGACAGACACATCGACCGCCTG
GCCAAACGCCAGCGCCCCGGCGAGCGGCTGGACCTGGCTATGCTGGCTGC
GATTCGCCGCGTTTACGGGCTACTTGCCAATACGGTGCGGTATCTGCAGTG
CGGCGGGTCGTGGCGGGAGGACTGGGGACAGCTTTCGGGGACGGCCGTGC
CGCCCCAGGGTGCCGAGCCCCAGAGCAACGCGGGCCCACGACCCCATATC
GGGGACACGTTATTTACCCTGTTTCGGGCCCCCGAGTTGCTGGCCCCCAAC
GGCGACCTGTATAACGTGTTTGCCTGGGCCTTGGACGTCTTGGCCAAACGC
CTCCGTTCCATGCACGTCTTTATCCTGGATTACGACCAATCGCCCGCCGGCT
GCCGGGACGCCCTGCTGCAACTTACCTCCGGGATGGTCCAGACCCACGTCA
CCACCCCCGGCTCCATACCGACGATATGCGACCTGGCGCGCACGTTTGCCC
GAGAGATGATCAGCGGAGCTAATGGCGTCATGGCCAAGTTGACCAGTGCC
GTTCCGGTGCTCACCGCGCGCGACGTCGCCGGAGCGGTCGAGTTCTGGACC
GACCGGCTCGGGTTCTCCCGGGACTTCGTGGAGGACGACTTCGCCGGTGTG
GTCCGGGACGACGTGACCCTGTTCATCAGCGCGGTCCAGGACCAGGTGGT
GCCGGACAACACCCTGGCCTGGGTGTGGGTGCGCGGCCTGGACGAGCTGT
ACGCCGAGTGGTCGGAGGTCGTGTCCACGAACTTCCGGGACGCCTCCGGG
CCGGCCATGACCGAGATCGGCGAGCAGCCGTGGGGGCGGGAGTTCGCCCT
GCGCGACCCGGCCGGCAACTGCGTGCACTTCGTGGCCGAGGAGCAGGACT
GACCGACGCCGACCAACACCGCCGGTCCGACGGCGGCCCACGGGTCCCAG
GGTCGACCTCGAGATCCTTAGGCCATTAAGGCCGGCCGCCTCGGCCCACTT
CGTGGGGTACCGAGCTCGAATTCACTGGCCGTCGTTTTACAACGTCGTGAC
TGGGAAAACCCTGGCGTTACCCAACTTAATCGCCTTGCAGCACATCCCCCT
TTCGCCAGCTGGCGTAATAGCGAAGAGGCCCGCACCGATCGCCCTTCCCAA
CAGTTGCGTGGCCGAGGAGCAGGACTGACACGTGCTACGAGATTTCGATT
CCACCGCCGCCTTCTATGAAAGGTTGGGCTTCGGAATCGTTTTCCGGGACG
CCGGCTGGATGATCCTCCAGCGCGGGGATCTCATGCTGGAGTTCTTCGCCC
ACCCCAACTTGTTTATTGCAGCTTATAATGGTTACAAATAAAGCAATAGCA
TCACAAATTTCACAAATAAAGCATTTTTTTCACTGCATTCTAGTTGTGGTTT

GTCCAAACTCATCAATGTATCTTATCATGTCTGTATACCGTCGACCTCTAGC
TAGAGCTTGGCGTAATCATGGTCATAGCTGTTTCCTGTGTGAAATTGTTATC
CGCTCACAATTCCACACAACATACGAGCCGGAAGCATAAAGTGTAAAGCC
TGGGGTGCCTAATGAGTGAGCTAACTCACATTAATTGCGTTGCGCTCACTG
CCCGCTTTCCAGTCGGGAAACCTGTCGTGCCAGCTGCATTAATGAATCGGC
CAACGCGCGGGGAGAGGCGGTTTGCGTATTGGGCGCTCTTCCGCTTCCTCG
CTCACTGACTCGCTGCGCTCGGTCGTTCGGCTGCGGCGAGCGGTATCAGCT
CACTCAAAGGCGGTAATACGGTTATCCACAGAATCAGGGGATAACGCAGG
AAAGAACATGTGAGCAAAAGGCCAGCAAAAGGCCAGGAACCGTAAAAAG
GCCGCGTTGCTGGCGTTTTTCCATAGGCTCCGCCCCCCTGACGAGCATCAC
AAAAATCGACGCTCAAGTCAGAGGTGGCGAAACCCGACAGGACTATAAAG
ATACCAGGCGTTTCCCCCTGGAAGCTCCCTCGTGCGCTCTCCTGTTCCGACC
CTGCCGCTTACCGGATACCTGTCCGCCTTTCTCCCTTCGGGAAGCGTGGCG
CTTTCTCAATGCTCACGCTGTAGGTATCTCAGTTCGGTGTAGGTCGTTCGCT
CCAAGCTGGGCTGTGTGCACGAACCCCCCGTTCAGCCCGACCGCTGCGCCT
TATCCGGTAACTATCGTCTTGAGTCCAACCCGGTAAGACACGACTTATCGC
CACTGGCAGCAGCCACTGGTAACAGGATTAGCAGAGCGAGGTATGTAGGC
GGTGCTACAGAGTTCTTGAAGTGGTGGCCTAACTACGGCTACACTAGAAG
GACAGTATTTGGTATCTGCGCTCTGCTGAAGCCAGTTACCTTCGGAAAAAG
AGTTGGTAGCTCTTGATCCGGCAAACAAACCACCGCTGGTAGCGGTGGTTT
TTTTGTTTGCAAGCAGCAGATTACGCGCAGAAAAAAAGGATCTCAAGAAG
ATCCTTTGATCTTTTCTACGGGGTCTGACGCTCAGTGGAACGAAAACTCAC
GTTAAGGGATTTTGGTCATGAGATTATCAAAAAGGATCTTCACCTAGATCC
TTTTAAATTAAAAATGAAGTTTTAAATCAATCTAAAGTATATATGAGTAAA
CTTGGTCTGACAGTTACCAATGCTTAATCAGTGAGGCACCTATCTCAGCGA
TCTGTCTATTTCGTTCATCCATAGTTGCCTGACTCCCCGTCGTGTAGATAAC
TACGATACGGGAGGGCTTACCATCTGGCCCCAGTGCTGCAATGATACCGCG
AGACCCACGCTCACCGGCTCCAGATTTATCAGCAATAAACCAGCCAGCCG
GAAGGGCCGAGCGCAGAAGTGGTCCTGCAACTTTATCCGCCTCCATCCAGT
CTATTAATTGTTGCCGGGAAGCTAGAGTAAGTAGTTCGCCAGTTAATAGTT
TGCGCAACGTTGTTGCCATTGCTACAGGCATCGTGGTGTCACGCTCGTCGT
TTGGTATGGCTTCATTCAGCTCCGGTTCCCAACGATCAAGGCGAGTTACAT

GATCCCCCATGTTGTGCAAAAAAGCGGTTAGCTCCTTCGGTCCTCCGATCG
TTGTCAGAAGTAAGTTGGCCGCAGTGTTATCACTCATGGTTATGGCAGCAC
TGCATAATTCTCTTACTGTCATGCCATCCGTAAGATGCTTTTCTGTGACTGG
TGAGTACTCAACCAAGTCATTCTGAGAATAGTGTATGCGGCGACCGAGTTG
CTCTTGCCCGGCGTCAATACGGGATAATACCGCGCCACATAGCAGAACTTT
AAAAGTGCTCATCATTGGAAAACGTTCTTCGGGGCGAAAACTCTCAAGGA
TCTTACCGCTGTTGAGATCCAGTTCGATGTAACCCACTCGTGCACCCAACT
GATCTTCAGCATCTTTTACTTTCACCAGCGTTTCTGGGTGAGCAAAAACAG
GAAGGCAAAATGCCGCAAAAAAGGGAATAAGGGCGACACGGAAATGTTG
AATACTCATACTCTTCCTTTTTCAATATTATTGAAGCATTTATCAGGGTTAT
TGTCTCATGAGCGGATACATATTTGAATGTATTTAGAAAAATAAACAAATA
GGGGTTCCGCGCACATTTCCCCGAAAAGTGCCACCTGACGTCGACGGATCG
GGAGATCTCCCGATCCCCTATGGTCGACTCTCAGTACAATCTGCTCTGATG
CCGCATAGTTAAGCCAGTATCTGCTCCCTGCTTGTGTGTTGGAGGTCGCTG
AGTAGTGCGCGAGCAAAATTTAAGCTACAACAAGGCAAGGCTTGACCGAC
AATTGCATGAAGAATCTGCTTAGGGTTAGGCGTTTTGCGCTGCTTCGCGAT
GTACGGGCCAGATATACGCGTTGACATTGATTATTGACTAGTTATTAATAG
TAATCAATTACGGGGTCATTAGTTCATAGCCCATATATGGAGTTCCGCGTT
ACATAACTTACGGTAAATGGCCCGCCTGGCTGACCGCCCAACGACCCCCGC
CCATTGACGTCAATAATGACGTATGTTCCCATAGTAACGCCAATAGGGACT
TTCCATTGACGTCAATGGGTGGACTATTTACGGTAAACTGCCCACTTGGCA
GTACATCAAGTGTATCATATGCCAAGTACGCCCCCTATTGACGTCAATGAC
GGTAAATGGCCCGCCTGGCATTATGCCCAGTACATGACCTTATGGGACTTT
CCTACTTGGCAGTACATCTACGTATTAGTCATCGCTATTACCATGGTGATG
CGGTTTTGGCAGTACATCAATGGGCGTGGATAGCGGTTTGACTCACGGGGA
TTTCCAAGTCTCCACCCCATTGACGTCAATGGGAGTTTGTTTTGGCACCAA
AATCAACGGGACTTTCCAAAATGTCGTAACAACTCCGCCCCATTGACGCAA
ATGGGCGGTAGGCGTGTACGGTGGGAGGTCTATATAAGCAGAGCTCTCTG
GCTAACTAGAGAACCCACTGCTTACTGGCTTATCGAAATTAATACGACTCA
CTATAGGGAGACCCAAGCTGGCTAGTGGATCCCCCGGGCTGCAGGAATTC
GATATCAAGCTTATCG

## Claims

1. A nucleic acid comprising the following contiguous elements arranged in the 5 prime to 3 prime direction;
a) a promoter;
b) at least two selectable markers;
c) a cloning site for receipt of a nucleic acid segment, said segment comprising a candidate regulatory RNA target sequence; and
d) a poly adenylation signal,
said elements arranged such that a transcript directed by said promoter comprises said at least two selectable markers, said candidate regulatory RNA target sequence, and said poly adenylation signal in that order.

2. A nucleic acid according to claim 1, wherein said candidate regulatory RNA target sequence is a candidate microRNA (miRNA) target sequence.

3. A nucleic acid according to claim 1 or claim 2, further comprising a stop codon located between said selectable markers and said cloning site.

4. A nucleic acid according to any preceding claim wherein at least two of said selectable markers are provided as an open reading frame encoding a single polypeptide comprising at least two selectable markers.

5. A nucleic acid according to claim 4 wherein said selectable markers comprise one marker for positive selection and one marker for negative selection.

6. A nucleic acid according to any preceding claim wherein said cloning site has inserted therein a nucleic acid segment comprising a 3 prime untranslated region (UTR) or a candidate 3 prime UTR.

7. A 3 prime UTR library, said library comprising a plurality of nucleic acids according to claim 6.

8. A 3 prime UTR library according to claim 7 wherein said candidate miRNA target sequences are comprised by cDNA's.

9. A cell comprising a nucleic acid according to any of claims 1 to 6 or a library according to any of claims 7 to 8.

10. A method of making a 3 prime UTR library comprising providing a nucleic acid according to any of claims 1 to 6, and inserting into said cloning site a nucleic acid comprising a 3 prime UTR or a candidate 3 prime UTR.

11. A method of making a 5 prime UTR library comprising providing a nucleic acid according to any of claims 1 to 6, and inserting into said cloning site a nucleic acid comprising a 5 prime UTR or a candidate 5 prime UTR.

12. A vector comprising a nucleic acid according to any of claims 1 to 6.

13. A method for identifying a miRNA target sequence comprising the steps of
(a) introducing a nucleic acid according to any of claims 1 to 6 comprising a candidate miRNA target sequence into a host cell;
(b) selecting host cell(s) expressing the first selectable marker of said nucleic acid;
(c) introducing at least one miRNA of interest to said host cell(s) of (b), and
(d) assaying for expression of the second selectable marker of said nucleic acid in the cells of (c),
wherein if the cells of (c) do not show expression of the second selectable marker then the candidate miRNA target sequence is identified as a miRNA target sequence.

14. A method for identifying an miRNA active against a miRNA target sequence comprising the steps of
(a) introducing a nucleic acid according to any of claims 1 to 6 comprising said miRNA target sequence into a host cell;
(b) selecting host cell(s) expressing the first selectable marker of said nucleic acid;
(c) introducing at least one miRNA of interest to said host cell(s) of (b), and
(d) assaying for expression of the second selectable marker of said nucleic acid in the cells of (c),
wherein if the cells of (c) do not show expression of the second selectable marker then the miRNA of interest is identified as an miRNA active against said miRNA target sequence.

15. A method for identifying an inhibitor of a regulatory RNA comprising the steps of
(a) introducing at least one regulatory RNA of interest into a host cell;
(b) introducing a nucleic acid according to any of claims 1 to 6 comprising a candidate regulatory RNA target sequence into said host cell;
(c) selecting host cell(s) which do not show expression the first selectable marker of said nucleic acid;
(d) introducing to said host cells a test substance or nucleic acid
(e) assaying for expression of the second said selectable marker in the cells of (d);
wherein if the cells of (d) show expression of the second selectable marker then the test substance or nucleic acid is identified as inhibiting said regulatory RNA.

16. A method for identifying a regulatory RNA target sequence comprising the steps of
(a) introducing a nucleic acid according to any of claims 1 to 6 comprising a candidate regulatory RNA target sequence into a host cell;
(b) selecting host cell(s) expressing the first selectable marker of said nucleic acid;
(c) introducing at least one regulatory RNA of interest to said host cell(s) of (b), and
(d) assaying for expression of the second selectable marker of said nucleic acid in the cells of (c),
wherein if the cells of (c) do not show expression of the second selectable marker then the candidate regulatory RNA target sequence is identified as a regulatory RNA target sequence.

17. A method according to claim 16 wherein said regulatory RNA is a siRNA and wherein said candidate regulatory RNA target sequence is a candidate siRNA target sequence.

## Patentansprüche

1. Nukleinsäure, welche folgende aufeinanderfolgenden Elemente umfasst, die in der 5'- bis3'-Richtung angeordnet sind;
a) einen Promotor;
b) zumindest zwei selektierbare Marker;
c) eine Klonierungsstelle zur Aufnahme eines Nukleinsäuresegments, wobei das Segment eine Kandidatenzielsequenz regulatorischer RNA umfasst; und
d) ein Polyadenylierungs-Signal,
wobei die Elemente so angeordnet sind, dass ein gesteuertes Transkript durch den Promotor zumindest zwei selektierbare Marker, die Kandidatenzielsequenz regulatorischer RNA, und das Polyadenylierungs-Signal in dieser Reihenfolge umfasst.

2. Nukleinsäure nach Anspruch 1, wobei die Kandidatenzielsequenz regulatorischer RNA eine Mikro-RNA-Kandidatenzielsequenz (miRNA) ist.

3. Nukleinsäure nach Anspruch 1 oder 2, welche außerdem ein Stopp-Codon umfasst, welches zwischen den selektierbaren Markern und der Klonierungsstelle angeordnet ist.

4. Nukleinsäure nach einem der vorgehenden Ansprüche, wobei zumindest zwei der selektierbaren Marker als ein offener Leserahmen bereitgestellt werden, der ein einzelnes Polypeptid codiert, welches zumindest zwei selektierbare Marker umfasst.

5. Nukleinsäure nach Anspruch 4, wobei die selektierbaren Marker einen Marker für positive Selektion und einen Marker für negative Selektion umfassen.

6. Nukleinsäure nach einem der vorhergehenden Ansprüche, wobei in die Klonierungsstelle ein Nukleinsäuresegment eingefügt ist, welches einen nichttranslatierten 3'- Bereich (UTR) oder einen 3'-Kandidaten-UTR umfasst.

7. 3'-UTR-Bibliothek, wobei die Bibliothek eine Vielzahl von Nukleinsäuren nach Anspruch 6 umfasst.

8. 3'-UTR-Bibliothek nach Anspruch 7, wobei die mi-RNA-Kandidatenzielsequenzen von cDNA's umfasst sind.

9. Zelle, welche eine Nukleinsäure nach einem der Ansprüche 1 bis 6 oder eine Bibliothek nach einem der Ansprüche 7 bis 8 umfasst.

10. Verfahren zur Herstellung einer 3'-UTR-Bibliothek, welches Bereitstellen einer Nukleinsäure nach einem der Ansprüche 1 bis 6 umfasst und Einfügen einer Nukleinsäure, welche einen 3'-UTRs oder einen 3'-Kandidaten-UTR in die Klonierungsstelle umfasst.

11. Verfahren zur Herstellung einer 5'-UTR-Bibliothek, welches Bereitstellen einer Nukleinsäure nach einem der Ansprüche 1 bis 6 und Einfügen einer Nukleinsäure umfasst, welche eine 5'-UTR oder eine 5'-Kandidaten-UTR umfasst.

12. Vektor, welcher eine Nukleinsäure nach einem der Ansprüche 1 bis 6 umfasst.

13. Verfahren zum Identifizieren einer miRNA-Zielsequenz, welches folgende Schritte umfasst:
(a) Einführen einer Nukleinsäure nach einem der Ansprüche 1 bis 6, welche eine miRNA-Kandidatenzielsequenz umfasst, in eine Wirtszelle;
(b) Auswählen einer Wirtszelle (Wirtszellen), welche den ersten selektierbaren Marker der Nukleinsäure exprimiert (exprimieren);
(c) Einführen zumindest einer miRNA von Interesse in die Wirtszelle (Wirtszellen) von (b), und
(d) Untersuchen auf Expression des zweiten selektierbaren Markers der Nukleinsäure in der Zelle (Zellen) von (c),
wobei, wenn die Zelle (Zellen) keine Expression des zweiten selektierbaren Markers zeigt (zeigen), die miRNA-Kandidatenzielsequenz als eine miRNA-Zielsequenz identifiziert wird.

14. Verfahren zum Identifizieren einer miRNA, welche gegen eine miRNA-Zielsequenz aktiv ist, welches folgende Schritte umfasst:
(a) Einführen einer Nukleinsäure nach einem der Ansprüche 1 bis 6, welche die miRNA-Zielsequenz umfasst, in eine Wirtszelle;
(b) Auswählen einer Wirtszelle (Wirtszellen), welche den ersten selektierbaren Marker der Nukleinsäure exprimieren;
(c) Einführen von zumindest einer miRNA von Interesse in die Wirtszelle (Wirtszellen) von (b), und
(d) Untersuchen auf Expression des zweiten selektierbaren Markers der Nukleinsäure in der Zelle (Zellen),
wobei, wenn die Zelle (Zellen) keine Expression des zweiten selektierbaren Markers zeigt (zeigen), die miRNA von Interesse als eine miRNA identifiziert wird, welches gegen die miRNA-Zielsequenz aktiv ist.

15. Verfahren zum Identifizieren eines Inhibitors einer regulatorischen RNA, welches folgende Schritte umfasst:
(a) Einführen zumindest einer regulatorischen RNA von Interesse in eine Wirtszelle;
(b) Einführen einer Nukleinsäure nach einem der Ansprüche 1 bis 6 , welche eine regulatorische RNA-Kandidatenzielsequenz umfasst, in die Wirtszelle;
(c) Auswählen einer Wirtszelle (Wirtszellen), welche keine Expression des ersten selektierbaren Markers der Nukleinsäure zeigt (zeigen);
(d) Einführen einer Testsubstanz oder Nukleinsäure in die Wirtszellen;
(e) Untersuchen, auf Expression, des zweiten selektierbaren Markers in den Zellen von (d);
wobei, wenn die Zellen von (d) eine Expression des zweiten selektierbaren Markers zeigen, die Testsubstanz oder Nukleinsäure als inhibierend für die regulatorische RNA identifiziert wird.

16. Verfahren zum Identifizieren einer regulatorischen RNA-Zielsequenz, welches folgende Schritte umfasst:
(a) Einführen einer Nukleinsäure nach einem der Ansprüche 1 bis 6, welche eine regulatorische RNA-Kandidatenzielsequenz umfasst, in die Wirtszelle;
(b) Auswählen einer Wirtszelle (Wirtszellen), welche den ersten selektierbaren Marker der Nukleinsäure exprimiert (exprimieren);
(c) Einführen zumindest einer regulatorischen RNA von Interesse in die Wirtszelle (Wirtszellen), und
(d) Untersuchen auf Expression des zweiten selektierbaren Markers der Nukleinsäure in der Zelle (Zellen),
wobei, wenn die Zelle (Zellen) keine Expression des zweiten selektierbaren Markers zeigt (zeigen), die regulatorische RNA-Kandidatenzielsequenz als eine regulatorische RNA-Zielsequenz identifiziert wird.

17. Verfahren nach Anspruch 16, wobei die regulatorische RNA eine siRNA ist und wobei die regulatorische RNA-Kandidatenzielsequenz eine siRNA-Kandidatenzielsequenz ist.

## Revendications

1. Acide nucléique comprenant les éléments contigus suivants agencés dans le sens 5 prime à 3 prime ;
a) un promoteur ;
b) au moins deux marqueurs sélectionnables ;
c) un site de clonage destiné à la réception d'un segment d'acide nucléique, ledit segment comprenant une séquence cible d'ARN de régulation candidate ; et
d) un signal de polyadénylation,
lesdits éléments étant agencés de telle sorte qu'un transcrit ordonné par ledit promoteur comprend lesdits au moins deux marqueurs sélectionnables, ladite séquence cible d'ARN de régulation candidate, et ledit signal de polyadénylation dans cet ordre.

2. Acide nucléique selon la revendication 1, dans lequel ladite séquence cible d'ARN de régulation candidate est une séquence cible de microARN (miARN) candidate.

3. Acide nucléique selon la revendication 1 ou la revendication 2, comprenant en outre un codon d'arrêt situé entre lesdits marqueurs sélectionnables et ledit site de clonage.

4. Acide nucléique selon l'une quelconque des revendications précédentes, dans lequel au moins deux marqueurs parmi lesdits marqueurs sélectionnables sont fournis en tant que cadre de lecture ouvert codant un seul polypeptide comprenant au moins deux marqueurs sélectionnables.

5. Acide nucléique selon la revendication 4, dans lequel lesdits marqueurs sélectionnables comprennent un marqueur pour une sélection positive et un marqueur pour une sélection négative.

6. Acide nucléique selon l'une quelconque des revendications précédentes, dans lequel ledit site de clonage comporte, inséré en son sein, un segment d'acide nucléique comprenant une région non traduite (UTR) en 3 prime ou une région UTR en 3 prime candidate.

7. Bibliothèque de régions UTR en 3 prime, ladite bibliothèque comprenant une pluralité d'acides nucléiques selon la revendication 6.

8. Bibliothèque de régions UTR en 3 prime selon la revendication 7, dans laquelle lesdites séquences cibles de miARN candidates comprennent plusieurs ADNc.

9. Cellule comprenant un acide nucléique selon l'une quelconque des revendications 1 à 6, ou une bibliothèque selon l'une quelconque des revendications 7 à 8.

10. Procédé de constitution d'une bibliothèque de régions UTR en 3 prime comprenant la fourniture d'un acide nucléique selon l'une quelconque des revendications 1 à 6, et l'insertion dans ledit site de clonage d'un acide nucléique comprenant une région UTR en 3 prime ou une région UTR en 3 prime candidate.

11. Procédé de constitution d'une bibliothèque de régions UTR en 5 prime comprenant la fourniture d'un acide nucléique selon l'une quelconque des revendications 1 à 6, et l'insertion dans ledit site de clonage d'un acide nucléique comprenant une région UTR en 5 prime ou une région UTR en 5 prime candidate.

12. Vecteur comprenant un acide nucléique selon l'une quelconque des revendications 1 à 6.

13. Procédé d'identification d'une séquence cible de miARN comprenant les étapes consistant à
(a) introduire un acide nucléique selon l'une quelconque des revendications 1 à 6 comprenant une séquence cible de miARN candidate dans une cellule hôte ;
(b) sélectionner une ou des cellules hôtes exprimant le premier marqueur sélectionnable dudit acide nucléique ;
(c) introduire au moins un miARN d'intérêt dans ladite ou lesdites cellules hôtes de l'étape (b), et
(d) rechercher l'expression du deuxième marqueur sélectionnable dudit acide nucléique dans les cellules de l'étape (c),
dans lequel si les cellules de l'étape (c) ne présentent pas l'expression du deuxième marqueur sélectionnable, alors la séquence cible de miARN candidate est identifiée comme une séquence cible de miARN.

14. Procédé d'identification d'un miARN actif par rapport à une séquence cible de miARN comprenant les étapes consistant à
(a) introduire un acide nucléique selon l'une quelconque des revendications 1 à 6 comprenant ladite séquence cible de miARN dans une cellule hôte ;
(b) sélectionner une ou des cellules hôtes exprimant le premier marqueur sélectionnable dudit acide nucléique ;
(c) introduire au moins un miARN d'intérêt dans ladite ou lesdites cellules hôtes de l'étape (b), et
(d) rechercher l'expression du deuxième marqueur sélectionnable dudit acide nucléique dans les cellules de l'étape (c),
dans lequel si les cellules de l'étape (c) ne présentent pas l'expression du deuxième marqueur sélectionnable, alors le miARN d'intérêt est identifié comme un miARN actif par rapport à ladite séquence cible de miARN.

15. Procédé d'identification d'un inhibiteur d'un ARN de régulation comprenant les étapes consistant à
(a) introduire au moins un ARN de régulation d'intérêt dans une cellule hôte ;
(b) introduire un acide nucléique selon l'une quelconque des revendications 1 à 6 comprenant une séquence cible d'ARN de régulation candidate dans ladite cellule hôte ;
(c) sélectionner une ou des cellules hôtes qui ne présentent pas l'expression du premier marqueur sélectionnable dudit acide nucléique ;
(d) introduire dans lesdites cellules hôtes une substance d'essai ou un acide nucléique
(e) rechercher l'expression du deuxième dit marqueur sélectionnable dans les cellules de l'étape (d) ;
dans lequel si les cellules de l'étape (d) présentent l'expression du deuxième marqueur sélectionnable, alors la substance d'essai ou l'acide nucléique est identifié comme inhibant ledit ARN de régulation.

16. Procédé d'identification d'une séquence cible d'ARN de régulation comprenant les étapes consistant à
(a) introduire un acide nucléique selon l'une quelconque des revendications 1 à 6 comprenant une séquence cible d'ARN de régulation candidate dans une cellule hôte ;
(b) sélectionner une ou des cellules hôtes exprimant le premier marqueur sélectionnable dudit acide nucléique ;
(c) introduire au moins un ARN de régulation d'intérêt dans ladite ou lesdites cellules hôtes de l'étape (b), et
(d) rechercher l'expression du deuxième marqueur sélectionnable dudit acide nucléique dans les cellules de l'étape (c),
dans lequel si les cellules de l'étape (c) ne présentent pas l'expression du deuxième marqueur sélectionnable, alors la séquence cible d'ARN de régulation candidate est identifiée comme une séquence cible d'ARN de régulation.

17. Procédé selon la revendication 16, dans lequel ledit ARN de régulation est un siARN et dans lequel ladite séquence cible d'ARN de régulation candidate est une séquence cible de siARN candidate.
